# FASCICULE DE BREVET EUROPEEN

(11) **EP 2 417 151 B1**
(45) Date de publication et mention de la délivrance du brevet: **14.09.2016**
(21) Numéro de dépôt: 10723209.2
(22) Date de dépôt: 06.04.2010
(51) Int. Cl.: C07K 5/12, A61P 33/02, A61P 33/06, A61P 33/00, A61K 38/12

(54) **PEPTIDES CYCLIQUES À ACTIVITÉ ANTIPARASITAIRE**
ZYKLISCHE PEPTIDE MIT ANTIPARASITÄRER WIRKUNG
CYCLIC PEPTIDES WITH AN ANTI-PARASITIC ACTIVITY

(30) Priorité: 06.04.2009 FR 0952244
(43) Date de publication de la demande: 15.02.2012
(73) Titulaire: Universite Joseph Fourier (Grenoble 1), 38041 Grenoble (FR); Centre National de la Recherche Scientifique, 75794 Paris Cedex 16 (FR); INSERM - Institut National de la Santé et de la Recherche Médicale, 75013 Paris (FR)
(72) Inventeur: WONG, Yung-Sing, F-38400 Saint Martin-d'Hères (FR); HAKIMI, Mohamed-Ali, F-38000 Grenoble (FR); BOUGDOUR, Alexandre, F-38000 Grenoble (FR); PELLOUX, Hervé, F-38190 Bernin (FR); MAUBON, Danièle, F-38330 Saint Ismier (FR)
(74) Mandataire: Delorme, Nicolas
(86) Numéro de dépôt international: PCT/FR2010/050658
(87) Numéro de publication internationale: WO 2010/116085

(56) Documents cités:
- WO-A-03/057722
- JP-A- 7 196 686
- QUICLET-SIRE BÉATRICE ET AL: "Powerful carbon-carbon bond forming reactions based on a novel radical exchange process." CHEMISTRY (WEINHEIM AN DER BERGSTRASSE, GERMANY) 7 AUG 2006, vol. 12, no. 23, 7 août 2006 (2006-08-07), pages 6002-6016, XP002543497 ISSN: 0947-6539
- QUICLET-SIRE B ET AL: "The Degenerative Radical Transfer of Xanthates and Related Derivatives: An Unusually Powerful Tool for the Creation of Carbon Carbon Bonds" TOPICS IN CURRENT CHEMISTRY, SPRINGER, BERLIN, DE, vol. 264, 1 janvier 2006 (2006-01-01), pages 201-236, XP008111064
- ANDREWS KATHERINE T ET AL: "Targeting histone deacetylase inhibitors for anti-malarial therapy." CURRENT TOPICS IN MEDICINAL CHEMISTRY 2009, vol. 9, no. 3, février 2009 (2009-02), pages 292-308, XP002543446 ISSN: 1873-4294
- MAUBON D., BOUGDOUR A., WONG Y.S., HAKIMI M.A., PELLOUX H.: "Drug inhibition of HDAC activity: effect on the parasite Toxoplasma gondii and chemotherapy perspectives" CLINICAL MICROBIOLOGY AND INFECTION, vol. 15, no. s4, 22 mai 2009 (2009-05-22), page S301, XP002543498

## Description

Le domaine de la présente invention est celui de la préparation d'un peptide cyclique à activité antiparasitaire. L'invention concerne également ce peptide en tant qu'agent antiparasitaire, par exemple dans le traitement de la toxoplasmose. L'invention concerne également l'utilisation de ce peptide cyclique pour traiter les organes *ex vivo* avant transplantation.

La toxoplasmose est une maladie parasitaire dont l'agent est un protozoaire intracellulaire opportuniste, *Toxoplasma gondii.* Les manifestations cliniques de la maladie chez les personnes immunocompétentes sont rares. Cependant, la toxoplasmose est de plus en plus associée chez ces personnes à des lymphadénopathies, fièvres, signes neurologiques (baisse de quotient intellectuel, diminution de performances psychomotrices), atteintes oculaires, infections disséminées sévères et même atteintes neurologiques ou psychiatriques comme la schizophrénie. Chez les patients immunodéprimés (par exemple sidéens, greffés, patients présentant des problèmes hématologiques) ou chez le foetus, *Toxoplasma gondii* se présente comme un pathogène opportuniste pouvant provoquer des atteintes graves.

Au cours de son cycle parasitaire, *Toxoplasma gondii* peut se présenter sous trois formes différentes : une forme tachyzoïte, une forme bradyzoïte et une forme oocyte. La forme chronique de la maladie est due à la forme bradyzoïte, contenue dans des kystes intracellulaires (5 à 70 µm) localisés notamment dans les cellules nerveuses et musculaires. Les kystes persistent dans ces tissus jusqu'à la mort de l'hôte. L'équilibre immunitaire hôte-parasite permet de les maintenir quiescents. Ces formes sont très résistantes et peuvent survivre plusieurs jours à température ambiante.

Un certain nombre de molécules (par exemple la pyriméthamine, la sulfadiazine et la sulfadoxine) sont présentes sur le marché. Cependant, ces molécules ont un effet contre la forme primaire tachyzoïte de la maladie. En effet, il n'existe actuellement aucun traitement contre la toxoplasmose chronique, aucun médicament n'étant à ce jour capable d'éliminer les kystes tissulaires. La seule molécule connue ayant un effet kysticide *in vitro* est l'atovaquone (US 5,641,769), l'activité kysticide de cette molécule *in vivo* n'ayant jamais été démontrée.

*Toxoplasma gondii* appartient au phylum des Apicomplexes (embranchement Apicomplexa) qui regroupe un grand nombre de parasites responsables de maladies telles que la malaria, la néosporose, la coccidiose et la cryptosporidiose. Les recherches visant à identifier de nouveaux principes actifs antiparasitaires ont permis d'identifier l'apicidine, tétrapeptide cyclique extrait du champignon *Fusarium pallidoroseum.* L'apicidine présente une certaine efficacité *in vivo* sur des souris infectées par la malaria de *Plasmodium berghei* (Darkin-Rattray et al. Proc. Natl. Acad. Sci. USA 1996 ; Singh et al., Tetrahedron Lett. 1996). Cette molécule aurait une action inhibitrice sur l'histone déacétylase (HDAC) de *Plasmodium berghei.*

Il existe différentes formes d'HDAC. Les inhibiteurs d'HDAC représentent une classe de plus de 8000 composés. Ils font aujourd'hui l'objet de recherche et développement, par exemple comme agents anticancéreux ou comme immunosuppresseur efficace et sélectif chez l'homme. La classe des inhibiteurs d'HDAC comprend notamment des dérivés naturels cycliques de type peptidique ayant montré une certaine efficacité. Parmi ceux-ci, on peut citer la chlamydocine, isolé du champignon *Diheterospora chlamydosporia* et qui a montré une activité anti-cancer *in vitro,* et les dérivés de la chlamydocine, isolés du champignon *Peniophora* sp. et qui provoquent l'arrêt du cycle cellulaire chez la plante (Tani et al. Phytochem. 2003).

Un certain nombre d'inconvénients est associé à l'utilisation des composés à visée thérapeutique humaine qui dérivent de matériel biologique, comme notamment l'apicidine et la chlamydocine. Notamment, il est impossible de maîtriser la composition exacte de l'extrait, par exemple la présence de molécules associées ayant potentiellement un effet biologique non maîtrisé. En outre, se pose le problème de l'accessibilité dudit matériel. Se pose également le problème du coût de préparation du composé actif.

Nishino et collaborateurs (Nishino *et al.,* Bioorg. Med. Chem, 2004) proposent plusieurs synthèses chimiques de dérivés de la chlamydocine. Chaque synthèse implique plusieurs réactions. Pour obtenir un dérivé, chaque synthèse met en oeuvre une approche synthétique différente, de sorte que les synthèses proposées présentent peu de flexibilité.

Un objet de la présente invention est de proposer un procédé de synthèse chimique visant à fournir des composés dont la pureté est contrôlée.

Un objet de l'invention est de proposer un procédé de préparation facile à mettre en oeuvre, à partir d'intermédiaires facilement accessibles et en utilisant des produits courants.

Un autre objet de la présente invention est de proposer un procédé présentant un coût de production attractif.

Un autre objet de la présente invention est de proposer un inhibiteur d'HDAC actif chez le parasite et ne présentant pas ou peu d'activité chez l'homme, ainsi qu'un procédé de synthèse d'un tel inhibiteur.

Un autre objet de la présente invention est de proposer un procédé de synthèse dont le composé de départ est un intermédiaire de synthèse.

Les inventeurs se sont donc intéressés à un nouveau procédé de préparation d'un tétrapeptide cyclique à activité antiparasitaire.

### Description de l'invention

### Procédé

L'invention concerne donc un procédé de préparation d'un peptide cyclique de formule (IV) décrite plus loin dans la description et appartenant à la formule (I) générale ci-après : 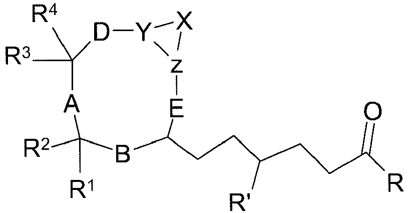 ledit procédé de l'invention s'inscrivant dans le procédé global comprenant les étapes suivantes :
on fait réagir un composé intermédiaire de formule (II) : 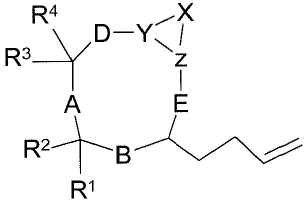 avec un carbonyle xanthate de formule (III) : 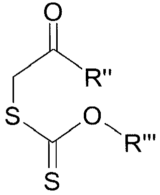 et, éventuellement dans une seconde étape, on procède à la déprotection du composé obtenu.

Dans le cadre de la formule générale (I) et du procédé global ci-dessus.

Les groupements des composés de formules (I) et (II) ont la signification suivante :
Y et Z, identiques ou différents, représentent un atome de carbone ou d'azote, Y et Z étant éventuellement reliés entre eux par une double liaison,
X représente 3 ou 4 atomes de carbone, azote et/ou oxygène, éventuellement substitués,
A représente un groupement R⁵ₐ-(N-CO)_{b}-R⁶_{c}
B représente un groupement R⁷_{d}-(N-CO)ₑ-R⁸_{f}
D représente un groupement R⁹_{g}-(N-CO)ₕ-R¹⁰ᵢ ou R⁹_{g}-(CO)ₕ-R¹⁰ᵢ
E représente un groupement R¹¹ⱼ-(N-CO)ₖ-R¹²ₗ
   dans lesquels R⁵, R⁶, R⁷, R⁸, R⁹, R¹⁰, R¹¹, R¹², identiques ou différents, représentent un, deux ou trois atomes de carbone, azote ou oxygène,
   dans lesquels a, b, c, d, e, f, g, h, i, j, k et l, identiques ou différents, sont choisis parmi 0, 1, 2 ou 3, sous réserve que le nombre d'atomes du cycle soit compris entre 12 et 16,
R représente H ; OH ; SH ; un groupe amine ; un groupe alkyle, haloalkyle ou hétéroalkyle contenant entre 1 et 10 atomes de carbone en chaîne linéaire ou ramifiée ; un groupe alcènyle ou alcynyle contenant entre 2 à 10 atomes de carbone en chaîne linéaire ou ramifiée ; un ou plusieurs groupes cycloalkyle, cycloacènyle ou cycloalcynyle contenant entre 3 à 10 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 10 atomes de carbone, les termes aryle et alkyle ayant les définitions ci-dessus ; un groupe alkoxy, thioalkyle, sulfonylalkyle, aminoalkyle contenant entre 1 et 10 atomes de carbone en chaîne linéaire ou ramifiée ; un groupe alkoxyalkyle, alkylthioalkyle, alkylsulfonylalkyle, alkylaminoalkyle contenant entre 1 et 30 atomes de carbone en chaîne linéaire ou ramifiée ; un ou plusieurs groupes hétérocycliques contenant entre 5 et 10 atomes de carbone par cycle ;
R' représente H ou un groupement xanthate ;
R¹, R², R³ et R⁴, identiques ou différents, représentent H ; OH, SH ; un groupe amine ; un groupe alkyle, haloalkyle ou hétéroalkyle contenant entre 1 et 30 atomes de carbone en chaîne linéaire ou ramifiée ; un groupe alcènyle ou alcynyle contenant entre 2 à 30 atomes de carbone en chaîne linéaire ou ramifiée ; un ou plusieurs groupes cycloalkyle, cycloacènyle ou cycloalcynyle contenant entre 3 à 30 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 30 atomes de carbone, les termes aryle et alkyle ayant les définitions ci-dessus ; un groupe alkoxy, thioalkyle, sulfonylalkyle, aminoalkyle contenant entre 1 et 30 atomes de carbone en chaîne linéaire ou ramifiée ; un groupe alkoxyalkyle, alkylthioalkyle, alkylsulfonylalkyle, alkylaminoalkyle contenant entre 1 et 30 atomes de carbone en chaîne linéaire ou ramifiée ; un ou plusieurs groupes hétérocycliques contenant entre 5 et 10 atomes par cycle, saturés ou insaturés, comprenant au moins un hétéroatome choisi parmi N, O et S, le ou lesdits groupes hétérocycliques pouvant être substitués, et étant liés directement ou indirectement par un radical alkylène bivalent au cycle dudit peptide.
   Les groupements du composé de formule (III) ont la signification suivante :
   R" représente H ; un groupe amine ; un groupe alkyle, haloalkyle ou hétéroalkyle contenant entre 1 et 10 atomes de carbone en chaîne linéaire ou ramifiée ; un groupe alcènyle ou alcynyle contenant entre 2 à 10 atomes de carbone en chaîne linéaire ou ramifiée ; un ou plusieurs groupes cycloalkyle, cycloacènyle ou cycloalcynyle contenant entre 3 à 10 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 10 atomes de carbone, les termes aryle et alkyle ayant les définitions ci-dessus ; un groupe alkoxy, thioalkyle, sulfonylalkyle, aminoalkyle contenant entre 1 et 10 atomes de carbone en chaîne linéaire ou ramifiée ; un groupe alkoxyalkyle, alkylthioalkyle, alkylsulfonylalkyle, alkylaminoalkyle contenant entre 1 et 10 atomes de carbone en chaîne linéaire ou ramifiée ; un ou plusieurs groupes hétérocycliques contenant entre 5 et 10 atomes de carbone par cycle ;
   R"' représente H ; un groupe alkyle ou haloalkyle contenant entre 1 et 10 atomes de carbone en chaîne linéaire ou ramifiée.

Selon l'invention, on prépare un peptide cyclique de formule (IV) : 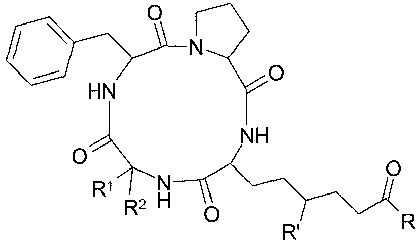 dans laquelle R, R¹ et R² sont tels que ci-dessus définis, et R' représente H ou un groupement xanthate de formule S-CS-O-R"' dans laquelle R"' représente H ; un groupe alkyle ou haloalkyle contenant entre 1 et 10 atomes de carbone en chaîne linéaire ou ramifiée,
procédé selon lequel on fait réagir un composé intermédiaire de formule (V) : 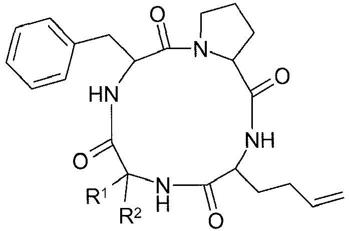 avec un carbonyle xanthate de formule (III) : 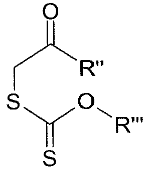 dans laquelle R" et R"' sont tels que ci-dessus définis,
et en ce que, éventuellement dans une seconde étape, on procède à la déprotection du composé obtenu.

Selon l'invention, le terme "alkyle" désigne un radical hydrocarboné linéaire ou ramifié de 1 à 30 atomes de carbone, tel que, à titre indicatif, méthyle, éthyle, propyle, isopropyle, butyle, tert-butyle, isobutyle, pentyle, hexyle, heptyle, octyle, nonyle, décyle, undécyle, dodécyle, tridécyle, tétradécyle, pentadécyle, hexadécyle, heptadécyle, octadécyle, nonadécyle ou icosyle. Le groupe alkyle ci-dessus défini peut comporter un ou plusieurs atomes d'halogène (fluor, chlore, brome ou iode). Dans ce cas, on parle de groupe "haloalkyle". Le groupe alkyle peut en outre comprendre des hétéroatomes choisis parmi P, O, N, S et Se. Dans ce cas, on parle de groupe "hétéroalkyle".

Par "alcényle", on entend une chaîne hydrocarbonée linéaire ou ramifiée de 2 à 30 atomes de carbone comprenant une ou plusieurs doubles liaisons. Des exemples de groupes alcényle sont les groupes alcényle portant une seule double liaison tels que -CH-CH=CH-CH₂, H₂C=CH- (vinyle) ou H₂C=CH-CH₂- (allyle).

Par "alcynyle", on entend une chaîne hydrocarbonée linéaire ou ramifiée de 2 à 30 atomes de carbone comprenant une ou plusieurs triples liaisons. Des exemples de groupes alcynyle sont les groupes alcynyle portant une seule triple liaison tel que -CH₂-C ≡CH.

Le terme "cycloalkyle" désigne des groupements hydrocarbonés saturés qui peuvent être mono- ou polycycliques et comprennent de 3 à 10 atomes de carbone. Il s'agit, par exemple, de groupements cycloalkyle monocycliques tels que cyclopropyle, cyclobutyle, cyclopentyle, cyclohexyle, cycloheptyle, cyclooctyle, cyclononyle, cyclodécyle, cycloundécyle et cyclododécyle.

Par "cycloalcényle", on entend selon l'invention un groupe dérivé d'un groupe cycloalkyle tel que défini ci-dessus, présentant une ou plusieurs doubles liaisons, par exemple deux doubles liaisons. Il s'agit par exemple du groupement cyclohexène (une double liaison) ou cyclopenta-1,3-diène (deux doubles liaisons).

Par "cycloalcynyle" entend selon l'invention un groupe dérivé d'un groupe cycloalkyle tel que défini ci-dessus, présentant une ou plusieurs triples liaisons, par exemple une triple liaison.

Le terme "aryle" représente un groupement hydrocarboné monocyclique ou polycyclique aromatique comprenant 3 à 10 atomes de carbone par cycle, tel que phényle ou naphtyle.

Le terme "hétéroaryle" désigne un groupe aromatique monocyclique ou polycyclique comprenant entre 3 et 10 atomes de carbone par cycle et comprenant 1, 2 ou 3 hétéroatomes endocycliques par cycle choisis parmi P, O, N, S et Se. Des exemples en sont les groupes furyle, thiényle, pyrrolyle, oxazolyle, isoxazolyle, thiazolyle, isothiazolyle, imidazolyle, pyrazolyle, oxadiazolyle, triazolyle, thiadiazolyle, pyridyle, pyridazinyle, pyrazinyle et triazinyle.

Par "alkaryle", on entend un groupe alkyle, substitué par un groupe aryle, ces deux groupes étant définis ci-dessus.

Par "aralkyle", on entend un groupe alkyle, substitué par un groupe aryle, ces deux groupes étant définis ci-dessus.

Par "alkoxy", on entend un groupe O-alkyle ayant de 1 à 30 atomes de carbone, notamment méthoxy, éthoxy, propoxy et butoxy. Par "alkoxyalkyle", on entend un groupe alkyle-O-alkyle ayant de 1 à 30 atomes de carbone. Les groupes "thioalkyle" ou "alkylthioalkyle", "sulfonylalkyle" ou "alkylsulfonylalkyle" et "aminoalkyle" ou "alkylaminoalkyle" comportent en outre respectivement un ou plusieurs atomes de soufre, un ou plusieurs groupes sulfonyl et une ou plusieurs fonctions amine.

Le terme "groupe hétérocyclique" désigne des cycles carbonés saturés ou insaturés, monocycliques ou polycycliques, présentant 1, 2 ou 3 hétéroatomes endocycliques choisis parmi P, O, N, S et Se. Ce sont généralement des dérivés des groupes hétéroaryles décrits ci-dessus. Des exemples d'hétérocycles insaturés sont dihydrofuryle, dihydrothiényle, dihydropyrrolyle, pyrrolinyle, oxazolinyle, thiazolinyle, imidazolinyle, pyrazolinyle, isoxazolinyle, isothiazolinyle, oxadiazolinyle, pyranyle et les dérivés mono- insaturés de la pipéridine, du dioxane, de la pipérazine, du trithiane, de la morpholine du dithiane, de la thiomorpholine, ainsi que tétrahydropyridazinyle, tétrahydropyrimidinyle, et tétrahydrotriazinyle.

Les groupes ci-dessus définis peuvent selon l'invention être substitués par un plusieurs groupes nitro, cyano, hydroxy, carboxy, carbonyl ou amino, par un ou plusieurs halogènes ou par une ou plusieurs fonctions nitrile, cyanhydrine, aldéhyde.

Selon un mode de réalisation de l'invention, le groupement R du composé de formule (IV) représente H ; OH ; SH ; un groupe alkyle ou haloalkyle contenant entre 1 et 10 atomes de carbone en chaîne linéaire ou ramifiée ; un ou plusieurs groupes aryle ou hétéroaryle contenant entre 3 à 10 atomes de carbone par cycle ; un groupe alkoxy, thioalkyle, sulfonylalkyle, aminoalkyle contenant entre 1 et 10 atomes de carbone en chaîne linéaire ou ramifiée ; un ou plusieurs groupes hétérocyclique contenant entre 5 et 10 atomes de carbone par cycle.

Selon un autre mode de réalisation de l'invention, le groupement R du composé de formule (IV) est choisi parmi CH₂Cl, CH₂Br, CF₃, OH, O-CH₂-C₆H₆, NHOH, CH₂-S-CS-O-CH₂CH₃, CH₂CH₃, CO-CH₂CO₂-CH₂CH₃, CHOH-CH₃, CH(OTBDMS)CH₃.

Selon un autre mode de réalisation de l'invention, le groupement R' du composé de formule (IV) représente H.

Selon un mode de réalisation de l'invention, le groupement R" du composé de formule (III) représente H ; un groupe alkyle ou haloalkyle contenant entre 1 et 10 atomes de carbone en chaîne linéaire ou ramifiée ; un ou plusieurs groupes aryle ou hétéroaryle contenant entre 3 à 10 atomes de carbone par cycle ; un groupe alkoxy, thioalkyle, sulfonylalkyle, aminoalkyle contenant entre 1 et 10 atomes de carbone en chaîne linéaire ou ramifiée ; un ou plusieurs groupes hétérocyclique contenant entre 5 et 10 atomes de carbone par cycle.

Selon un mode de réalisation de l'invention, le groupement R" du composé de formule (III) est choisi parmi CH₂Cl, CH₂Br, CF₃, O-CH₂-C₆H₆, CH₂CH₃, COCH₂CO₂Et, CH(OTBDMS)CH₃.

Selon un mode de réalisation de l'invention, un example de peptide cyclique est le composé de formule (IV'f) : 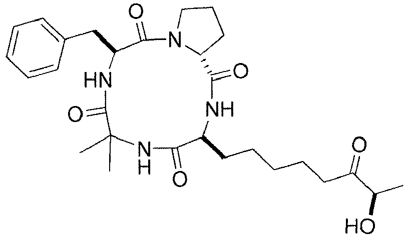 La présente invention concerne également un composé intermédiaire de formule (V) dans laquelle R¹ et R² sont tels que définis précédemment. Un composé intermédiaire préféré selon l'invention répond à la formule (V) dans laquelle au moins l'un de R¹ et R² représente CH₂C≡CH. Lorsque seul l'un de R¹ et R² représente CH₂C≡CH, l'autre représente avantageusement H.

### Peptides cycliques à activité antiparasitaire

L'invention concerne également un peptide cyclique à activité antiparasitaire de formule (IV) et appartenant à la formula (I) générale ci-après 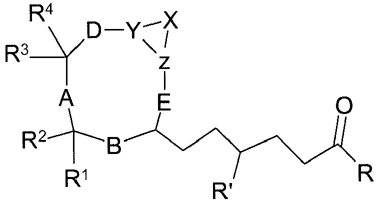 dans laquelle X, Y, Z, A, B, D, E, R, R', R¹, R², R³ et R⁴ sont tel que ci-dessus définis.

Le peptide cyclique de l'invention et pouvant être obtenu selon la présente invention est celui représenté par la formule (IV) : 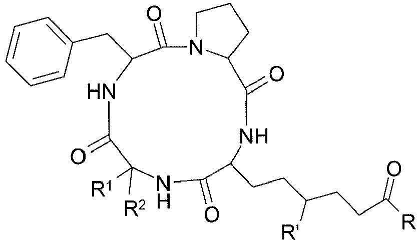 dans laquelle R, R', R¹ et R² sont tels que ci-dessus définis en référence à la formule (IV).

Un autre exemple de peptide cyclique pouvant être obtenu selon la présente invention est celui représenté par la formule (IV) : 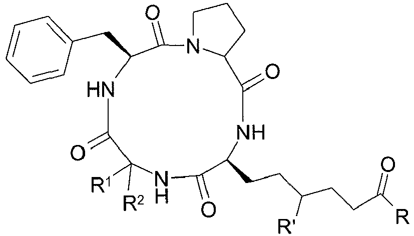 dans laquelle R et R' sont tels que ci-dessus définis,
et au moins l'un de R¹ et R² représente CH₂C≡CH, l'autre de R¹ et R² représentant H, le cas échéant.

### Selon un mode de réalisation, R' représente H.

Selon un autre mode de réalisation, combiné ou non aux modes de réalisation précités, R représente CHOH-CH₃.

Selon un mode de réalisation de l'invention, le peptide cyclique à activité antiparasitaire est le composé de formule (IV'f) :

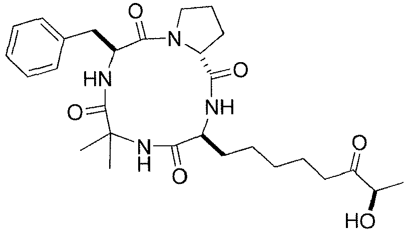

Selon un autre mode de réalisation, l'invention concerne un peptide cyclique ayant un effet contre la toxoplasmose, par exemple contre la forme chronique de la toxoplasmose.

### Utilisations

L'invention concerne également un peptide cyclique de formule (IV) pour son utilisation comme agent antiparasitaire, en particulier dans le traitement de la malaria, la toxoplasmose, la coccidiose, la cryptosporidiose ou la néosporose.

L'invention concerne aussi un peptide cyclique de l'invention pour un traitement anti-cancer. L'analogie des peptides de l'invention avec les dérivés naturels cycliques de type peptidique connus pour leur activité anti-cancer, permet d'envisager des propriétés identiques pour les peptides de l'invention. Dans la partie des exemples qui suit, ces propriétés sont d'ailleurs démontrées pour un composé préféré de l'invention.

L'invention concerne également l'utilisation du peptide cyclique de formule (IV) pour traiter un organe *ex vivo* avant transplantation.

### Exemples

### 1. Préparation du composé intermédiaire de formule (VI), notamment du composé (VII)

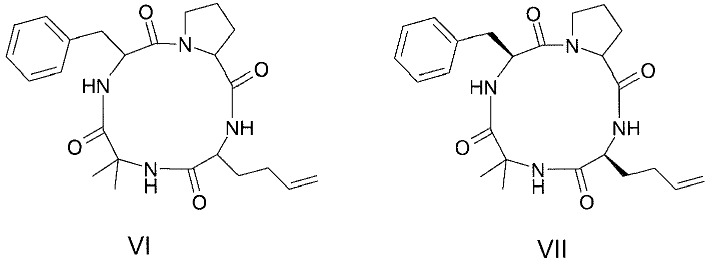

Le composé intermédiaire (VI) a été obtenu selon le procédé ci-dessous décrit.

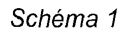

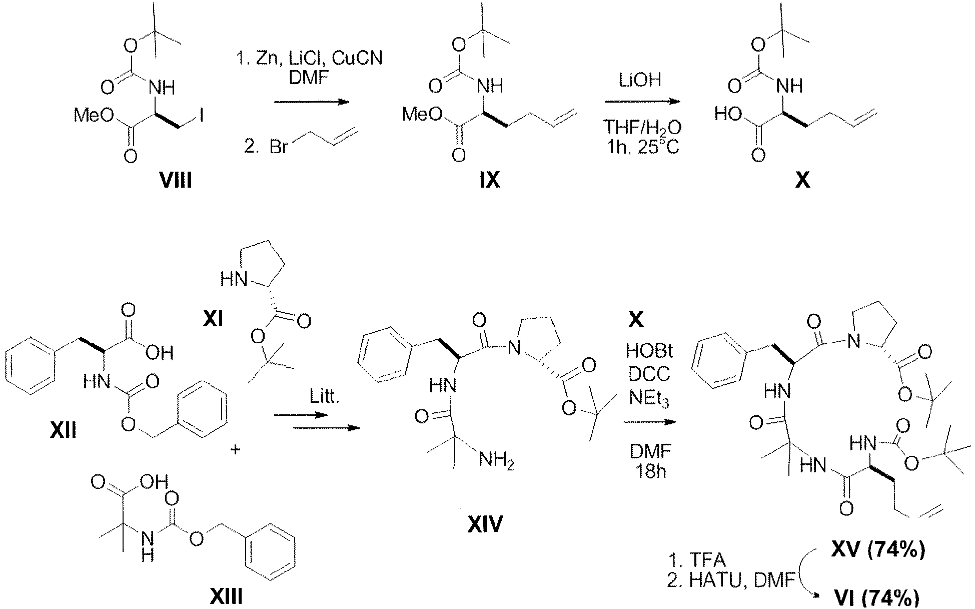

Brièvement, un acide aminé (X) a été synthétisé à partir de (VIII) (Jackson *et al.,* Org. Synth. 2005) suivant la méthode décrite par Kiyota et collaborateurs (Shimasaki *et al.,* Tetrahedron 2006). Le tripeptide (XIV) est obtenu à partir des trois acides aminés commerciaux (XI), (XII) et (XIII) selon une synthèse peptidique en phase liquide (Bhuiyan et al., Bioorg. Med. Chem. 2006). Le composé (XIV) réagit avec le composé (X) pour donner le tétrapeptide (XV), qui conduit au composé intermédiaire (VII).

### Préparation du composé (S)-méthyl 2-(tert-butoxycarbonylamino)hex-5-énoate (IX)

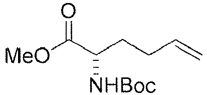

Dans une solution de DMF anhydre (28 mL) contenant de la poudre de zinc (2 .48 g, 37.9 mmol) est injectée du 1,2-dibromoéthane (0.17 mL) sous argon. La solution est agitée pendant 20 min à température ambiante. Du TMSCI (50 µL) est injecté et la solution est chauffée sous agitation à 60°C pendant 30 min. Le (*R*)-méthyl 2-(*tert-*butoxycarbonylamino)-3-iodopropanoate (VIII) (2.0 g, 6.08 mmol) dans du DMF (8 mL) est additionné goutte à goutte. La solution est ensuite agitée pendant 20 min à 60°C. Du LiCl (587 mg, 13.8 mmol) et du CuCN (619 mg, 6.9 mmol) dans du DMF (6.5 mL) sont injectés à - 55°C et la solution est placée à 0°C pendant 10 min. Cette dernière est replacée à - 55°C et du bromure d'allyle (1.05 mL, 12 mmol) est injecté. Après 5 min, la solution est placée à 0°C et agitée pendant 2 h à cette température. Le zinc qui n'a pas réagit est éliminé par filtration sur célite et le filtrat est traité par une solution saturée de NH₄Cl. Le produit est extrait avec de l'acétate d'éthyle (EtOAc). Les phases organiques réunies sont lavées avec une solution saturée en NaCl, puis séchées sur MgSO₄ et évaporées. Après flash chromatographie (gel de silice, 8% EtOAc/cyclohexane), le produit (IX) (1.38 g, 93%) est isolé sous forme d'huile incolore. R*_{f}* = 0.25 (10% EtOAc/cyclohexane) ; [α]^{D} = +20.5 (c = 1, CHCl₃) ; IR vₘₐₓ (film, CH₂Cl₂) 3358, 2978, 1745, 1716, 1518, 1453, 1366, 1249, 1163, 914 cm⁻¹; RMN ¹H (400 MHz, CDCl₃) δ ppm 1.45 (s, 9H), 1.74 (m, 1H), 1.90 (m, 1H), 2.12 (m, 2H), 3.74 (s, 3H), 4.32 (m, 1 H), 5.00 (bd, *J =* 10.3 Hz, 1 H), 5.05 (bd, *J =* 17.0, 1H), 5.18 (bd, *J* = 7.2 Hz, NH), 5.79 (m, 1H) ; RMN ¹³C (100 MHz, CDCl₃) δ ppm 28.4 (3×CH₃), 29.6 (CH₂), 32.0 (CH₂), 52.3 (CH₃), 53.0 (CH), 79.8 (C), 115.7 (CH₂), 137.0 (CH), 155.4 (C), 173.4 (C) ; Masse (ESI+) *m*/*z* (%) 266 (100) [*M+*Na]⁺, 210 (43), 166 (38).

### Préparation de l'acide (S)-2-(tert-butoxycarbonylamino)hex-5-énoique (X)

Dans une solution de THF/H₂O (1 :1, 35 mL) contenant le produit (IX) (1 g, 4.1 mmol) est additionné en une portion LiOH.H₂O (432 mg, 10.3 mmol). Le mélange est agité pendant 1 h à température ambiante. Le solvant est ensuite évaporé puis une solution 5% H₃PO₄ dans l'eau est additionnée jusqu'à l'obtention d'un pH = 3. Le produit est ensuite extrait avec de l'EtOAc, séché (MgSO₄), puis évaporé. Après purification, le produit (X) (840 mg, 89%) est isolé sous forme de laque incolore.

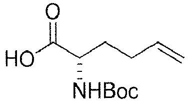

### Préparation du (3S,9S,14aR)-9-benzyl-3-(but-3-ényl)-6,6-diméthyldécahydropyrrolo [1,2-a][1,4,7,10]tétraazacyclododécine-1,4,7,10-tétraone (VII)

A une solution de tripeptide H-Aib-L-Phe-D-Pro-O*t*-Bu (XIV) (1.38 g, 3.42 mmol) et de méthyl (*S*)-2-(benzyloxycarbonylamino)-5-hexenoate (X) (784 mg, 3.42 mmol) dans du DMF anhydre (7 ml) sont additionnés de l'HOBt.H₂O (525 mg, 3.45 mmol), du DCC (850 mg, 4.12 mmol) et de la triéthyl amine (0.5 mL) sous argon. La solution est agitée à température ambiante pendant 18 h. Le DMF est évaporé et le résidu est dilué dans de l'EtOAc. Cette phase est lavée avec une solution aqueuse d'acide citrique 10%, une solution aqueuse de sodium carbonate 4% et une solution aqueuse saturée NaCl. La phase organique est séchée (MgSO₄) et évaporée. Après flash chromatographie (gel de silice, 1.5 % MeOH/CH₂Cl₂), le tétrapeptide (XV) (1.58 g, 75%) est obtenu sous forme de laque incolore. R*_{f}* = 0.09 (2% MeOH/CH₂Cl₂); LRMS (ESI+) *m*/*z* (%) 637 (100) [*M*+Na]⁺, 615 (20) [*M*+H]⁺; SMHR (ESI+) : *m*/*z* calculée pour C₃₃H₅₀N₄O₇Na 637.3577, trouvée 637.3567. Le tétrapeptide (XV) (1.57 g, 2.55 mmol) est dissous dans de l'acide trifluoroacétique (7 mL) à 0°C et la solution est agitée pendant 3 h à cette température. Après évaporation, le produit est précipité dans de l'éther sec pour donner après filtration un sel déprotégé (1.08 g, 74%). Ce dernier (900 mg, 1.57 mmol) est repris dans du DMF anhydre (140 mL). De l'HATU (666 mg, 1.75 mmol) et du diisopropyl éthyl amine (0.81 mL) sont ajoutés en cinq portions sur un intervalle de 30 min sous forte agitation. L'agitation est poursuivie pendant 1 h à température ambiante. Le DMF est évaporé et l'EtOAc est ajouté. Après lavage avec une solution aqueuse d'acide citrique 10%, suivi d'une solution aqueuse de sodium carbonate 4%, la phase organique est séchée (MgSO₄) et évaporée. Après flash chromatographie (gel de silice, 1% MeOH/CH₂Cl₂), le cyclotétrapeptide (VII) (512 mg, 74%) est isolé sous forme de solide blanc.
R_{f} = 0.17 (40% EtOAc/cyclohexane) ; [α]^{D} = -110 (c = 0.84, CHCl₃) ; IR vₘₐₓ (thin film, CH₂Cl₂) 3303, 2930, 1678, 1663, 1630, 1663, 1529, 1427, 1251, 1181, 913 cm⁻¹; RMN ¹H (400 MHz, CDCl₃) δ ppm 1.31 (s, 3H), 1.67-1.82 (m, 6H), 1.92 (m, 1H), 2.09 (m, 2H), 2.16 (m, 1 H), 2.30 (m, 1H), 2.94 (dd, *J* = 13.5, 5.8 Hz, 1H), 3.19-3.28 (m, 2H), 3.86 (m, 1 H), 4.30 (ddd, *J* = 10.1, 7.5, 7.5 Hz, 1 H), 4.66 (m, 1 H), 4.97 (m, 1 H), 5.00 (m, 1 H), 5.17 (ddd, *J* = 10.1, 10.0, 5.8 Hz, 1H), 5.78 (ddt, *J* = 16.8, 10.2, 6.6 Hz, 1H), 6.18 (s, NH), 7.16-7.28 (m, 5H, NH), 7.61 (d, *J* = 10.1 Hz, NH) ; RMN ¹³C (100 MHz, CDCl₃) *δ* ppm 23.4 (CH₃), 24.6 (CH₂), 24.8 (CH₂), 26.3 (CH₃), 28.2 (CH₂), 29.6 (CH₂), 35.7 (CH₂), 46.7 (CH₂), 53.2 (CH), 53.7 (CH), 57.6 (CH), 58.5 (C), 115.5 (CH₂), 126.5 (CH), 128.4 (2xCH), 128.9 (2×CH), 136.9 (C, CH), 171.5 (C), 172.6 (C), 174.2 (C), 175.4 (C) ; Masse (ESI+) *mlz* (%) 463 (100) [*M*+Na]⁺; SMHR (ESI+) : *m*/*z* calculée pour C₂₄H₃₂N₄O₄Na 463.2321, trouvée 463.2316.

### 2. Préparation des carbonyles xanthates de formule (III)

Tel que cela est représenté dans le schéma 2 ci-dessous, les produits (XVI_{a-g}) réagissent avec du potassium d'éthyl xanthate pour donner les carbonyles xanthates (III_{a-g}). 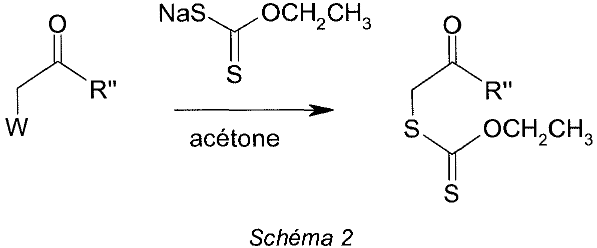
XVIa : W=Cl ; R"= CH₂Cl IIIa : R"= CH₂Cl (Litt)
XVIb : W=Br ; R"= CF₃ IIIb : R"= CF₃ (Litt)
XVIc : W=Cl ; R"= OBn IIIc : R"= OBn (68%; purifié par distillation)
XVId : W=Br ; R"= CH₂CH₃ IIId : R"= CH₂CH₃ (63%; purifié par distillation)
XVIe : W=Cl ; R"= COCH₂COEt IIIe : R"= COCH₂COEt (67%; purifié par distillation)
XVIF: W=Cl ; R"= CH(OTBDMS)CH₃ (Configuration S)
IIIf : R"= CH₂Cl (96%; purifié par chromatographie)
XVIf : W=Cl ; R"= CH(OTBDMS)CH₃ (Configuration R)
IIIg : R"= CH₂Cl (96%; purifié par chromatographie)

### Procédure type pour la préparation des composés intermédiaires (III), exemple avec le benzyl 2-(éthoxycarbonothioylthio)acétate (IIIc)

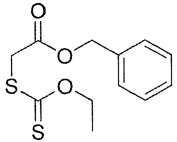

A une solution de benzyl 2-chloroacetate 1c (2.76 g, 15 mmol) dans l'acétone (50 mL) est ajouté à 0°C une solution d'éthyl xanthate de potassium (2.6 g, 16 mmol) dans l'acétone (50 mL). La solution est agitée pendant 4 h à température ambiante. L'acétone est évaporée et CH₂Cl₂ est ajouté. La phase organique est lavée par une solution saturée de NaCl. La phase organique est séchée (MgSO₄) et évaporée. Le produit est distillé par Kugelrohr (13 mbar, 180-200°C) pour donner (IIIc) (2.74 g, 68%) sous forme d'une huile jaune claire. Dans le cas de (IIIf) et (IIIg), une chromatographie classique est effectuée.
R_{f}= 0.21 (5% EtOAc/cyclohexane) ; IR vₘₐₓ (film) 2982, 1740, 1615, 1498, 1455, 1376, 1232, 1150 cm⁻¹; RMN ¹H (400 MHz, CDCl₃) δ ppm 1.35 (t, *J* = 7.1 Hz, 3H), 3.93 (s, 2H), 4.58 (q, *J* = 7.1 Hz, 2H), 5.18 (s, 2H), 7.32-7.36 (m, 5H). RMN ¹³C (100 MHz, CDCl₃) δ ppm 13.7 (CH₃), 37.9 (CH₂), 67.6 (CH₂), 70.7 (CH₂), 128.3 (CH), 128.4 (2xCH), 128.6 (2xCH), 135.3 (C), 167.8 (C), 212.4 (C) ; Masse (ESI+) *m*/*z* (%) 293 (100) [*M*+Na]⁺.

### Préparation du O-éthyl S-2-oxobutyl carbonodithioate (IIId)

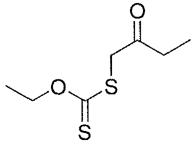

Produit (IIId), purifié par distillation (Kugelrohr, 2.2 mbar, 100°C), Rdt% = 63% (huile jaune claire) ; IR vₘₐₓ (film) 2980, 1729, 1717, 1457, 1376, 1224, 1113, 1052 cm⁻¹; RMN ¹H (400 MHz, CDCl₃) δ ppm 1.11 (t, *J* = 7.1 Hz, 3H), 1.42 (t, *J* = 7.1 Hz, 3H), 2.65 (q, *J* = 7.1 Hz, 2H), 4.00 (s, 2H), 4.63 (q, *J* = 7.1 Hz, 2H) ; RMN ¹³C (100 MHz, CDCl₃) δ ppm 7.97 (CH₃), 13.9 (CH₃), 35.4 (CH₂), 45.3 (CH₂), 71.0 (CH₂), 204.1 (C), 213.6 (C) ; Masse (ESI+) *m*/*z (%)* 215 (100) [M+Na]⁺.

### Préparation de l'éthyl 4-(éthoxycarbonothioylthio)-3-oxobutanoate (IIIe)

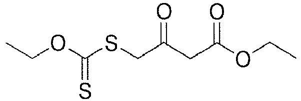

Produit (IIIe), purifié par distillation (Kugelrohr, 2.2 mbar, 150°C), Rdt% = 67% (huile jaune claire) ; IR vₘₐₓ (film) 2983,1745,1616,1376,1225,1113,1048 cm⁻¹; RMN ¹H (400 MHz, CDCl₃) δ ppm 1.30 (t, *J* = 7.1 Hz, 3H), 1.42 (t, *J* = 7.1 Hz, 3H), 3.65 (s, 2H), 4.12 (s, 2H), 4.20 (q, *J* = 7.1 Hz, 2H), 4.64 (q, *J* = 7.1 Hz, 2H) ; RMN ¹³C (100 MHz, CDCl₃) δ ppm 13.8 (CH₃), 14.2 (CH₃), 45.5 (CH₂), 48.3 (CH₂), 61.8 (CH₂), 71.2 (CH₂), 166.9 (C), 196.3 (C), 213.0 (C) ; Masse (ESI+) *m*/*z* (%) 273 (100) [M+Na]⁺.

### Préparation du (R)-S-3-(tert-butyldiméthylsilyloxy)-2-oxobutyl O-éthyl carbonodithioate (IIIf)

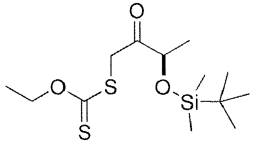

Produit (IIIf), purifié par flash chromatography (5% éther/cyclohexane), Rdt% = 96% (huile jaune claire) ; R*_{f}* = 0.25 (4% éther/cyclohexane) ; [α]^{D} = -7.3 (c = 1.15, CHCl₃) ; IR vₘₐₓ (film) 2955, 2931, 1735, 1728, 1363, 1224, 1112, 1051, 836 cm⁻¹; RMN ¹H (400 MHz, CDCl₃) δ ppm 0.13 (s, 3H), 0.14 (s, 3H), 0.95 (s, 9H), 1.37 (d, *J* = 6.8 Hz, 3H), 1.41 (t, *J* = 7.1 Hz, 3H), 4.28 (d, *J* = 17.9 Hz, 1 H), 4.35 (d, *J* = 17.9 Hz, 1 H), 4.62 (q, *J* = 7.1 Hz, 2H) ; RMN¹³ C (100 MHz, CDCl₃) δ ppm -4.9 (CH₃), -4.4 (CH₃), 13.9 (CH₃), 18.1 (C), 21.0 (CH₃), 25.9 (2×CH₃), 42.8 (CH₂), 70.6 (CH₂), 75.0 (CH), 205.7 (C), 213.6 (C) ; Masse (ESI+) *m*/*z (%)* 345 (100) [*M*+Na]⁺, 323 (5).

### Préparation du (S)-S-3-(tert-butyldiméthylsilyloxy)-2-oxobutyl O-éthyl carbonodithioate (IIIg)

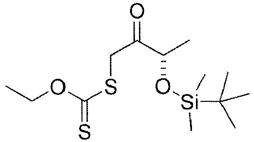

Produit (IIIg), purifié par flash chromatographie (5% éther/cyclohexane), Rdt% = 94% (huile jaune claire) ; R*_{f}* = 0.25 (4% éther/cyclohexane) ; [α]^{D} = +7.4 (c = 1.2, CHCl₃) ; IR vₘₐₓ (film) 2955, 2931, 1735, 1728, 1363, 1224, 1112, 1051, 836 cm⁻¹; RMN ¹H (400 MHz, CDCl₃) δ ppm 0.13 (s, 3H), 0.14 (s, 3H), 0.95 (s, 9H), 1.37 (d, *J* = 6.8 Hz, 3H), 1.41 (t, *J* = 7.1 Hz, 3H), 4.28 (d, *J* = 17.9 Hz, 1 H), 4.35 (d, *J* = 17.9 Hz, 1 H), 4.62 (q, *J* = 7.1 Hz, 2H) ; RMN¹³ C (100 MHz, CDCl₃) δ ppm -4.9 (CH₃), -4.4 (CH₃), 13.9 (CH₃), 18.1 (C), 21.0 (CH₃), 25.9 (2×CH₃), 42.8 (CH₂), 70.6 (CH₂), 75.0 (CH), 205.7 (C), 213.6 (C) ; Masse (ESI+) *m*/*z (%)* 345 (100) [*M*+Na]⁺, 323 (8).

### 3. Procédé de préparation de peptides cycliques de formule (IV) :

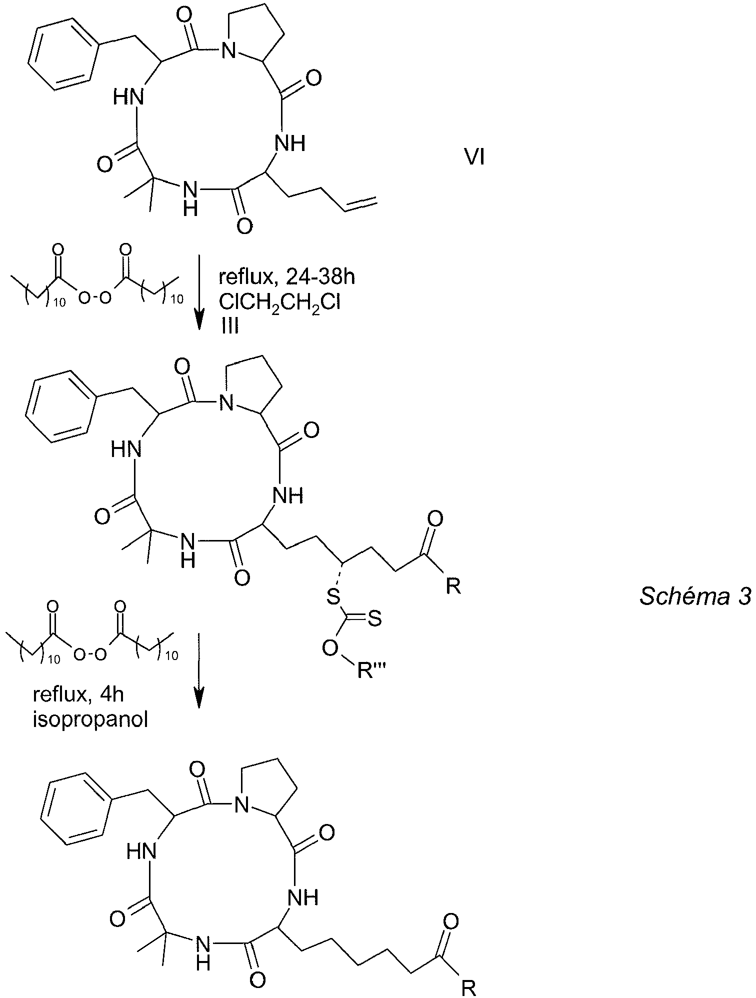

A partir du composé intermédiaire de formule (VI), les conditions radicalaires ont été appliquées avec différents carbonyles xanthates (III) pour installer le groupement carbonyle en position 8 (voir le schéma 3 ci-dessus). Après chauffage dans le 1,2-dichloroéthane en présence de carbonyle xanthate (III) et de lauroyl peroxide (initiateur radicalaire), le composé (IV) : dans lequel R¹ et R² sont CH₃ et R' est un xanthate sont obtenus avec des rendements allant de 65 à 95%. La fonction xanthate résultante est ensuite éliminée en présence de lauroyl peroxide chauffé dans l'isopropanol pour donner les peptides cycliques de formule (IV') dans lesquels R¹ et R² sont CH₃ et R' représente H (60-75%).

Ces deux étapes peuvent être également réalisées en une seule étape (en « one pot »). Dans ce cas, on évapore le 1,2-dichloroéthane pour le substituer avec de l'isopropanol puis on ajoute du lauroyl peroxide. Dans ces conditions, les peptides cycliques de formule (IV') dans lesquels R' représente H sont obtenus à partir du composé intermédiaire (VI) avec des rendements globaux allant de 48 à 52%.

### Procédé de préparation de peptides cycliques de formule (IV), exemple avec le S-1-((3S,9S,14aR)-9-benzyl-6,6-diméthyl-1,4,7,10-tétraoxotétradécahydropyrrolo[1,2-a][1,4,7,10] tétraazacyclododécin-3-yl)-7-chloro-6-oxoheptan-3-yl O-éthyl carbonodithioate (IVa)

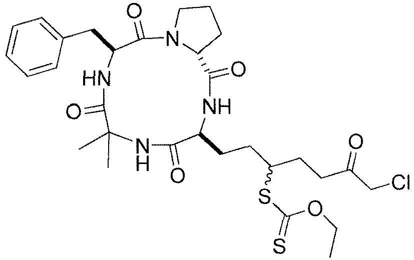

Le peptide cyclique (IVa) correspond au peptide cyclique de formule (IV) dans lequel R représente CH₂Cl, R¹ et R² sont CH₃ et R' est un xanthate.

Le cyclotétrapeptide (VI) (75 mg, 0.17 mmol) et le carbonyl xanthate (IIIa) (72 mg, 0.34 mmol) dans du 1,2-dichloroéthane (0.3 mL) sont chauffés à reflux pendant 30 min sous argon. 7 mg de dilauroyl peroxide (DLP) sont ajoutés tous les 2 h (5 à 7 fois en tout). Au bout de 8 à 18 h à reflux (réaction suivie par CCM), le solvant est évaporé et le produit directement purifié par flash chromatographie sur gel de silice (pour (IVa), 40% EtOAc/cyclohexane) pour donner (IVa) (72 mg, 65%) sous forme d'une laque incolore. R_{f} = 0.16 (40% EtOAc/cyclohexane) ; IR vₘₐₓ (film) 3302, 2925, 1734, 1678, 1663, 1628, 1525, 1435, 1390, 1217, 1111, 1048 cm⁻¹; RMN ¹H (400 MHz, CDCl₃) δ ppm 1.35 (s, 3H), 1.43 (t, *J =* 7.1 Hz, 3H), 1.62-1.96 (m, 10H), 2.07-2.23 (m, 2H), 2.32 (m, 1 H), 2.76 (m, 2H), 2.95 (dd, *J* = 13.5, 5.7 Hz, 1H), 3.23 (m, 1 H), 3.26 (dd, *J* = 13.5, 10.2 Hz, 1 H), 3.76 (m, 1H), 3.86 (m, 1H), 4.08 (s, 2H), 4.22 (m, 1H), 4.65 (q, *J* = 7.1 Hz, 2H), 4.66 (m, 1H), 5.17 (m, 1H), 5.92 (s, NH), 7.15 (bd, *J* = 10.1 Hz, NH), 7.19-7.32 (m, 5H), 7.47 (bd, *J* = 10.1 Hz, NH) ; RMN ¹³C (100 MHz, CDCl₃) δ ppm 14.0 (CH₃), 23.7 (CH₃), 24.9 (CH₂), 25.2 (CH₂), 26.7 (CH₃), 28.0 (CH₂), 29.9 (CH₂), 31.1 (CH₂), 36.0 (CH₂), 37.0 (CH₂), 47.2 (CH₂), 48.4 (CH₂), 50.7 (CH), 53.6 (CH), 54.3 (CH), 58.0 (CH), 59.0 (C), 70.5 (CH₂), 126.9 (CH), 128.8 (2×CH), 129.2 (2×CH), 137.2 (C), 172.1 (C), 173.1 (C), 174.1 (C), 175.7 (C), 202.0 (C), 214.3 (C) ; Masse (ESI+) *m*/*z (%)* 675 (100) [*M*+Na]⁺, 653 (32); SMHR (ESI+) : *m*/*z* calculée pour C₃₀H₄₁N₄O₆ClNaS₂ 675.2054, trouvée 675.2053.

### Préparation du benzyl 6-((3S,9S,14aR)-9-benzyl-6,6-dimethyl-1,4,7,10-tetraoxotetradecahydropyrrolo[1,2-a][1,4,7,10]tetraazacyclododecin-3-yl)-4-(ethoxycarbonothioylthio)hexanoate (IVc)

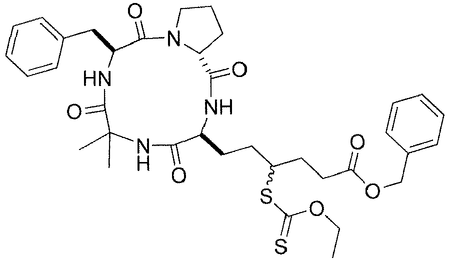

Suivant la procédure décrite ci-dessus, produit (IVc), purifié par flash chromatographie (1% MeOH/CH₂Cl₂), Rdt% = 90% ; R_{f} = 0.06 (1% MeOH/CH₂Cl₂) ; IR vₘₐₓ (film) 3308, 2940, 1734, 1681, 1663, 1629, 1525, 1454, 1436, 1215, 1111, 1049, 910 cm⁻¹; RMN ¹H (400 MHz, CDCl₃) δ ppm 1.34 (s, 3H), 1.41 (t, *J* = 7.1 Hz, 3H), 1.65-1.81 (m, 8H), 1.86-2.00 (m, 2H), 2.05-2.26 (m, 2H), 2.32 (m, 1H), 2.52 (m, 2H), 2.95 (dd, *J* = 13.5, 5.7 Hz, 1 H), 3.23 (m, 1 H), 3.26 (dd, *J* = 13.5, 10.1 Hz, 1 H), 3.79 (m, 1 H), 3.86 (m, 1 H), 4.21 (m, 1 H), 4.62 (q, *J* = 7.1 Hz, 2H), 4.66 (m, 1 H), 5.12 (s, 2H), 5.17 (m, 1 H), 5.92 (s, NH), 7.13 (bd, *J =* 10.2 Hz, NH), 7.19-7.39 (m, 10H), 7.47 (bd, *J* = 10.2 Hz, NH) ; RMN ¹³C (100 MHz, CDCl₃) δ ppm 13.8 (CH₃), 23.5 (CH₃), 24.7 (CH₂), 25.0 (CH₂), 26.2 (CH₂), 26.5 (CH₃), 29.2 (CH₂), 30.6 (CH₂), 31.5 (CH₂), 35.8 (CH₂), 47.0 (CH₂), 50.4 (CH), 53.4 (CH), 54.1 (CH), 57.8 (CH), 58.8 (C), 66.4 (CH₂), 70.1 (CH₂), 126.7 (CH), 128.3 (2×CH), 128.6 (2×CH ), 128.7 (2×CH), 129.0 (2xCH), 135.8 (C), 137.0 (C), 171.9 (C), 172.7 (C), 172.8 (C), 173.9 (C), 175.6 (C), 213.9 (C) ; Masse (ESI+) *m*/*z* (%) 733 (100) [M+Na]⁺, 711 (46), 463 (25); SMHR (ESI+) : *m*/*z* calculée pour C₃₆H₄₆N₄O₇NaS₂ 733.2706, trouvée 733.2709.

### Préparation du S-(R)-1-((3S,9S,14aR)-9-benzyl-6,6-diméthyl-1,4,7,10-tétraoxo tétradécahydropyrrolo[1,2-a][1,4,7,10]tétraazacyclododécin-3-yl)-7-(tert-butyldiméthyl silyloxy)-6-oxooctan-3-yl O-éthyl carbonodithioate (IVf)

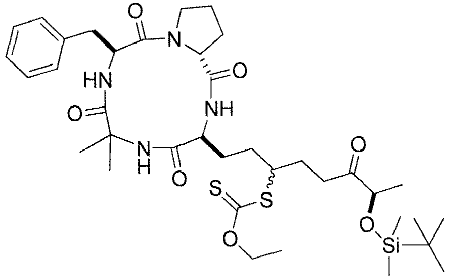

Suivant la procédure décrite ci-dessus, produit (IVf) purifié par flash chromatographie (0.8% MeOH/CH₂Cl₂), Rdt% = 96% ; R*_{f}*= 0.1 (1% MeOH/CH₂Cl₂) ; RMN ¹H (400 MHz, CDCl₃) δ ppm 0.07 (s, 6H), 0.91 (s, 9H), 1.28 (m, 3H), 1.34 (s, 3H), 1.42 (t, *J* = 7.1 Hz, 3H), 1.62-1.88 (m, 9H), 1.92-2.11 (m, 2H), 2.18 (m, 1H), 2.33 (m, 1 H), 2.76 (m, 2H), 2.96 (dd, *J* = 13.5, 5.7 Hz, 1 H), 3.23 (m, 1 H), 3.27 (dd, *J* = 13.5, 10.1 Hz, 1 H), 3.77 (m, 1 H), 3.86 (m, 1H), 4.14 (q, *J* = 6.7 Hz, 1 H), 4.22 (m, 1 H), 4.63 (q, *J* = 7.1 Hz, 2H), 4.66 (m, 1 H), 5.17 (m, 1H), 5.96 (s, NH), 7.15 (d, *J*= 10.1 Hz, NH), 7.18-7.30 (m, 5H), 7.51 (bd, *J* = 10.1 Hz, NH) ; RMN ¹³C (100 MHz, CDCl₃) δ ppm -4.8 (CH₃), -4.5 (CH₃), 14.0 (CH₃), 18.2 (CH₃), 23.7 (CH₃), 24.9 (CH₂), 25.2 (CH₂), 25.9 (3×CH₃), 26.4 (CH₂), 26.7 (CH₂), 27.6 (CH₂), 31.4 (CH₂), 34.5 (CH₂), 36.0 (CH₂), 47.2 (CH₂), 51.1 (CH), 53.6 (CH), 54.3 (CH), 58.0 (CH), 59.0 (C), 70.2 (CH₂), 75.0 (CH), 126.9 (CH), 128.8 (2×CH), 129.2 (2×CH), 137.2 (C), 172.1 (C), 173.0 (C), 174.1 (C), 175.8 (C), 213.5 (C), 214.4 (C) ; Masse (ESI+) *m*/*z* (%) 785 (90) [*M*+Na]⁺, 763 (70), 413 (100) ; SMHR (ESI+) : *m*/*z* calculée pour C₃₇H₅₈N₄O₇NaSiS₂ 785.3414, trouvée 785.3407.

### Préparation du S-(S)-1-((3S,9S,14aR)-9-benzyl-6,6-diméthyl-1,4,7,10-tétraoxo tétradécahydropyrrolo[1,2-a][1,4,7,10]tétraazacyclododécin-3-yl)-7-(tert-butyl diméthylsilyloxy)-6-oxooctan-3-yl O-éthyl carbonodithioate (IVg)

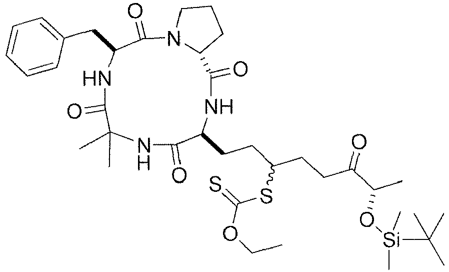

Suivant la procédure décrite ci-dessus, produit (IVg), purifié par flash chromatographie (0.8% MeOH/CH₂Cl₂), Rdt% = 95% ; R_{f}= 0.1 (1% MeOH/CH₂Cl₂) ; RMN ¹H (400 MHz, CDCl₃) δ ppm 0.07 (s, 6H), 0.91 (s, 9H), 1.26 (m, 3H), 1.35 (s, 3H), 1.42 (t, *J* = 7.1 Hz, 3H), 1.60-1.86 (m, 9H), 1.90-2.08 (m, 2H), 2.17 (m, 1H), 2.33 (m, 1H), 2.76 (m, 2H), 2.95 (dd, *J* = 13.5, 5.7 Hz, 1H), 3.23 (m, 1 H), 3.26 (dd, *J* = 13.5, 10.1 Hz, 1H), 3.77 (m, 1H), 3.86 (m, 1 H), 4.13 (q, *J* = 6.7 Hz, 1 H), 4.24 (m, 1 H), 4.63 (q, *J* = 7.1 Hz, 2H), 4.67 (m, 1H), 5.17 (m, 1H), 6.00 (bs, NH), 7.15 (d, *J* = 10.1 Hz, NH), 7.18-7.30 (m, 5H), 7.50 (bd, *J* = 10.1 Hz, NH) ; RMN ¹³C (100 MHz, CDCl₃) δ ppm -4.9 (CH₃), -4.5 (CH₃), 14.0 (CH₃), 18.2 (CH₃), 23.7 (CH₃), 24.9 (CH₂), 25.2 (CH₂), 25.9 (3×CH₃), 26.4 (CH₂), 26.6 (CH₃), 27.6 (CH₂), 31.2 (CH₂), 34.4 (CH₂), 36.0 (CH₂), 47.1 (CH₂), 51.0 (CH), 53.6 (CH), 54.5 (CH), 58.0 (CH), 59.0 (C), 70.2 (CH₂), 75.0 (CH), 126.9 (CH), 128.8 (2×CH), 129.2 (2xCH), 137.2 (C), 172.0 (C), 173.0 (C), 174.1 (C), 175.8 (C), 213.6 (C), 214.3 (C) ; Masse (ESI+) *m*/*z* (%) 785 (100) [M+Na]⁺, 763 (30) ; SMHR (ESI+) : *m*/*z* calculée pour C₃₇H₅₈N₄O₇NaSiS₂ 785.3414, trouvée 785.3431.

### Procédure générale pour obtenir (IV') à partir de (IV) (approche en deux étapes), exemple avec le (3S,9S,14aR)-9-benzyl-3-(7-chloro-6-oxoheptyl)-6,6-diméthyl décahydropyrrolo[1,2-a][1,4,7,10]tétraazacyclododécine-1,4,7,10-tétraone (IV'a)

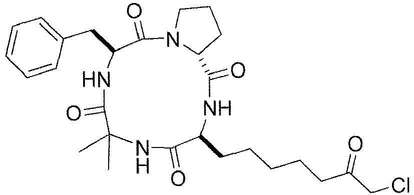

Le peptide cyclique (IV'a) correspond au peptide cyclique de formule (IV) dans lequel R représente CH₂Cl, R¹ et R² sont CH₃ et R' représente H. En outre, deux liaisons ont une configuration définie.
Le peptide cyclique (IVa) (12 mg, 0.018 mmol) dans de l'isopropanol (1 mL) est chauffé à reflux pendant 5 h en présence de DLP (11 mg, 0.027 mmol). Après évaporation et flash chromatographie (gel de silice, 30% EtOAc/cyclohexane), le produit (IV'a) (9 mg, 92%) est obtenu sous forme de laque incolore. R*_{f}* = 0.07 (40% EtOAc/cyclohexane) ; [α]^{D} = -80 (c = 1.0, CHCl₃) ; IR vₘₐₓ (film) 3304, 2928, 1733, 1678, 1663, 1628, 1525, 1434, 1390, 1179 cm⁻¹; RMN ¹H (400 MHz, CDCl₃) δ ppm 1.29-1.34 (m, 7H), 1.63 (m, 2H), 1.69-1.87 (m, 7H), 2.17 (m, 1 H), 2.32 (m, 1 H), 2.56 (t, *J* = 7.3 Hz, 2H), 2.95 (dd, *J* = 13.5, 5.7 Hz, 1H), 3.21 (dd, *J* = 10.0, 7.0 Hz, 1H), 3.26 (dd, *J* = 13.5, 10.0 Hz, 1 H), 3.86 (m, 1 H), 4.07 (s, 2H), 4.19 (ddd, *J =* 10.2, 7.6, 7.6 Hz, 1 H), 4.67 (m, 1H), 5.16 (ddd, *J* = 10.1, 10.0, 5.7 Hz, 1H), 5.95 (s, NH), 7.11 (bd, *J* = 10.1Hz, NH), 7.19-7.29 (m, 5H), 7.51 (bd, *J* = 10.1 Hz, NH); RMN ¹³C (100 MHz, CDCl₃) δ ppm 23.4 (CH₂), 23.7 (CH₃), 24.9 (CH₂), 25.2 (CH₂), 25.4 (CH₂), 26.7 (CH₃), 28.8 (CH₂), 28.9 (CH₂), 36.0 (CH₂), 39.6 (CH₂), 47.1 (CH₂), 48.3 (CH₂), 53.6 (CH), 54.4 (CH), 57.9 (CH), 59.0 (C), 126.9 (CH), 128.8 (2×CH), 129.2 (2×CH), 137.2 (C), 172.0 (C), 173.0 (C), 175.0 (C),175.8 (C), 202.7 (C); Masse (ESI+) *m*/*z* (%) 555 (100) [M+Na]⁺, 533 (10); SMHR (ESI+) : *m*/*z* calculée pour C₂₇H₃₇N₄O₅ClNaS₂ 555.2350, trouvée 555.2343.

### Préparation du Benzyl 6-(3S,9S,14aR)-9-benzyl-6,6-diméthyl-1,4,7,10-tétraoxo tétradécahydropyrrolo[1,2-a][1,4,7,10]tétraazacyclododécin-3-yl)hexanoate (IV'c)

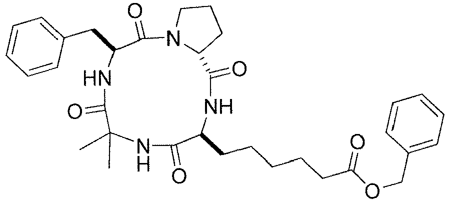

A partir de (IVc), le produit (IV'c) est obtenu après purification par flash chromatographie (1% MeOH/CH₂Cl₂), Rdt% = 69%; R_{f} = 0.17 (40% EtOAc/cyclohexane) ; [α]^{D} = -75 (c = 1.4, CHCl₃) ; IR vₘₐₓ (film) 3309, 2934, 1735, 1678, 1663, 1629, 1525, 1424, 1257, 1173 cm⁻¹; RMN ¹H (400 MHz, CDCl₃) δ ppm 1.25-1.40 (m, 7H), 1.65 (m, 2H), 1.72 (m, 1H), 1.77-187 (m, 6H), 2.17 (m, 1H), 2.29 (m, 1 H), 2.35 (t, *J* = 7.5 Hz, 2H), 2.95 (dd, *J* = 13.5, 5.7 Hz, 1H), 3.19-3.29 (m, 2H), 3.86 (ddd, *J* = 10.3, 8.6, 4.6 Hz, 1 H), 4.18 (ddd, *J* = 10.2, 7.6, 7.6 Hz, 1 H), 4.65 (m, 1H), 5.11 (s, 2H), 5.16 (ddd, *J =* 10.2, 10.1, 5.7, 1H), 5.90 (s, NH), 7.09 (bd, *J =* 10.2 Hz, NH), 7.17-7.40 (m, 10H), 7.51 (bd, *J* = 10.3 Hz, NH) ; RMN ¹³C (100 MHz, CDCl₃) δ ppm 23.7 (CH₃), 24.86 (CH₂), 24.93 (CH₂), 25.2 (CH₂), 25.4 (CH₂), 26.7 (CH₃), 28.9 (2×CH₂), 34.3 (CH₂), 36.0 (CH₂), 47.2 (CH₂), 53.6 (CH), 54.5 (CH), 58.0 (CH), 59.0 (C), 66.3 (CH₂), 126.9 (CH), 128.4 (2×CH), 128.7 (2×CH ), 128.8 (2×CH), 129.2 (2×CH), 136.2 (C), 137.2 (C), 172.0 (C), 173.0 (C), 173.6 (C), 174.5 (C), 175.8 (C); Masse (ESI+) *mlz* (%) 613 (100) [*M*+Na]⁺, 591 (18) ; SMHR (ESI+) : *m*/*z* calculée pour C₃₃H₄₂N₄O₆Na 613.3002, trouvée 613.2991.

### Préparation du (3S,9S,14aR)-9-benzyl-3-((S)-7-(tert-butyldiméthylsilyloxy)-6-oxooctyl)-6,6-diméthyldécahydropyrrolo[1,2-a][1,4,7,10]tétraazacyclododécine-1,4,7,10-tétraone (IV'g)

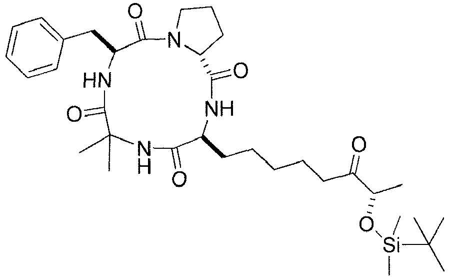

A partir de (IVg), le produit (IV'g) est obtenu après purification par flash chromatographie (0.8% MeOH/CH₂Cl₂), Rdt% = 59%; RMN ¹H (400 MHz, CDCl₃) δ ppm 0.07 (s, 6H), 0.91 (s, 9H), 1.25-1.34 (m, 9H), 1.55 (m, 2H), 1.63 (m, 2H), 1.76 (m, 1H), 1.77 (s, 3H), 2.17 (m, 1H), 2.32 (m, 1 H), 2.47-2.64 (m, 2H), 2.95 (dd, *J* = 13.5, 5.7 Hz, 1H), 3.21 (m, 1H), 3.26 (dd, *J* = 13.5, 10.1 Hz, 1H), 3.86 (m, 1H), 4.13 (q, *J* = 6.8 Hz, 1H), 4.20 (ddd, *J* = 10.1, 7.6, 7.6 Hz, 1H), 4.67 (bd, *J* = 7.6 Hz, H1), 5.17 (m, 1H), 6.11 (bs, NH), 7.15 (d, *J* = 10.1 Hz, NH), 7.18-7.29 (m, 5H), 7.57 (bd, *J* = 10.1 Hz, NH); RMN ¹³C (100 MHz, CDCl₃) δ ppm -4.9 (CH₃), -4.5 (CH₃), 21.0 (CH₃), 22.9 (CH₂), 23.1 (CH₂), 23.8 (CH₃), 24.9 (CH₂), 25.2 (CH₂), 25.9 (3×CH₃), 26.6 (CH₃), 29.8 (CH₂), 29.9 (CH₂), 36.0 (CH₂), 36.9 (CH₂), 47.1 (CH₂), 53.6 (CH), 54.6 (CH), 58.0 (CH), 58.9 (C), 75.1 (CH), 126.9 (CH), 128.8 (2×CH), 129.2 (2×CH), 137.2 (C), 172.0 (C), 173.0 (C), 174.5 (C), 175.8 (C), 214.3 (C); Masse (ESI+) *m*/*z* (%) 665 (100) [M+Na]⁺, 643 (30).

### Préparation du (3S,9S,14aR)-9-benzyl-3-((R)-7-(tert-butyldiméthylsilyloxy)-6-oxooctyl)-6,6-diméthyldécahydropyrrolo[1,2-a][1,4,7,10]tétraazacyclododécine-1,4,7,10-tétraone (IV'f)

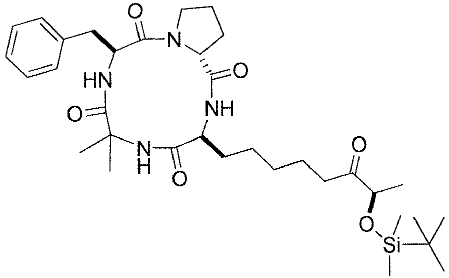

A partir de (IVf), le produit (IV'f) est obtenu après purification par flash chromatographie (0.8% MeOH/CH₂Cl₂), Rdt% = 60% ; [α]^{D} = -47 (c = 2.3, CHCl₃) ; RMN ¹H (400 MHz, CDCl₃) δ ppm 0.07 (s, 6H), 0.91 (s, 9H), 1.26-1.34 (m, 7H), 1.34 (s, 3H), 1.55-1.73 (m, 4H), 1.75 (m, 1H), 1.77 (s, 3H), 2.17 (m, 1H), 2.32 (m, 1H), 2.47-2.64 (m, 2H), 2.95 (dd, *J* = 13.5, 5.7 Hz, 1 H), 3.21 (m, 1 H), 3.26 (dd, *J* = 13.5, 10.1 Hz, 1H), 3.86 (m, 1H), 4.13 (q, *J* = 6.8 Hz, 1H), 4.19 (ddd, *J* = 10.1, 7.6, 7.6 Hz, 1H), 4.66 (bd, *J* = 7.8 Hz, 1H), 5.16 (m, 1H), 6.03 (s, NH), 7.12 (d, *J* = 10.1 Hz, NH), 7.18-7.29 (m, 5H), 7.55 (bd, *J* = 10.1 Hz, NH) ; RMN ¹³C (100 MHz, CDCl₃) δ ppm -4.8 (CH₃), -4.5 (CH₃), 21.1 (CH₃), 22.9 (CH₂), 23.2 (CH₂), 23.8 (CH₃), 25.0 (CH₂), 25.2 (CH₂), 26.0 (3×CH₃), 26.7 (CH₂), 29.8 (CH₂), 29.9 (CH₂), 36.0 (CH₂), 36.9 (CH₂), 47.2 (CH₂), 53.7 (CH), 54.6 (CH), 58.0 (CH), 59.0 (C), 75.1 (CH), 126.9 (CH), 128.8 (2×CH), 129.3 (2×CH), 137.3 (C), 172.0 (C), 173.0 (C), 174.6 (C), 175.9 (C), 214.4 (C); Masse (ESI+) *m*/*z* (%) 665 (17) [*M*+Na]⁺, 551 (100); SMHR (ESI+) : *m*/*z* calculée pour C₃₄H₅₄N₄OₑNaSᵢ 665.3710, trouvée 665.3717.

Selon un autre mode de réalisation (schéma 4), l'alcène terminal du composé intermédiaire (VI) est substitué différemment en effectuant un autre type de réaction radicalaire. Par exemple, la réaction avec l'acide thio-acétique (XVII) (par exemple R^{a} = un groupe alkyle contenant entre 1 et 10 atomes de carbone en chaîne linéaire ou ramifiée, notamment CH₃) sur l'intermédiaire (VI) en présence d'un initiateur de radical (AIBN) conduit à la formation du thio-ester tétrapeptide cyclique (XVIII) (91%).

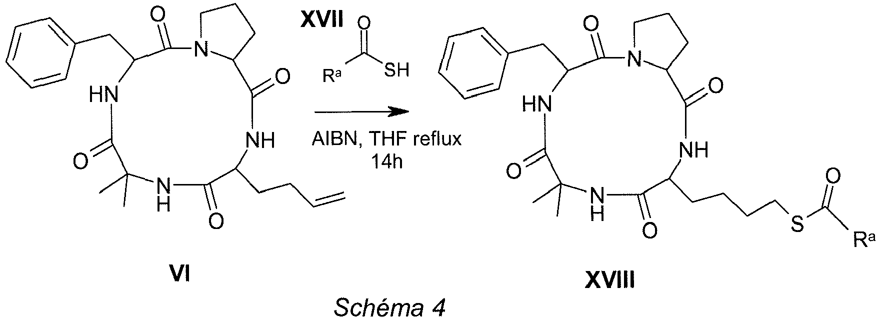

### Préparation du S-4-((3S,9S,14aR)-9-benzyl-6,6-diméthyl-1,4,7,10-tétraoxotétradécahydro pyrrolo[1,2-a][1,4,7,10]tétraazacyclododécin-3-yl)butyl éthanethioate (XVIIIa)

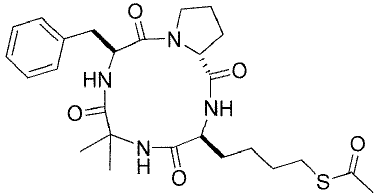

Le composé (VI) (32 mg, 0.073 mmol) dans du THF anhydre (10 mL) est ajouté de l'acide thioacetique (22 mg, 0.29 mmol). La solution est chauffée à reflux pendant 30 min sous argon. Une quantité catalytique d'AIBN est ajoutée et la réaction est agitée à reflux pendant 16 h. Après évaporation du solvant, le résidu est directement purifié par flash chromatographie (1% MeOH/CH₂Cl₂) pour donner le produit (XVIIIa) (34 mg, 91%).
R*_{f}* = 0.12 (1% MeOH/CH₂Cl₂) ; IR vₘₐₓ (film) 3307, 2934, 1684, 1630, 1528, 1428, 1274, 1187, 915 cm⁻¹; RMN ¹H (400 MHz, CDCl₃) δ ppm 1.34 (s, 3H), 1.39 (m, 2H), 1.53-1.71 (m, 4H), 1.72 (m, 5H), 2.11-2.22 (m, 2H), 2.32 (s, 3H), 2.85 (t, *J* = 7.2 Hz, 2H), 2.95 (dd, *J* = 13.4, 5.6 Hz, 1 H), 3.23 (m, 1 H), 3.26 (m, 1 H), 3.86 (m, 1 H), 4.21 (m, 1 H), 4.68 (m, 1 H), 5.17 (s, 1H), 6.02 (s, NH), 7.13 (d, *J* = 10.1 Hz, NH), 7.19-7.29 (m, 5H), 7.53 (d, *J* = 10.1 Hz, NH) ; RMN ¹³C (100 MHz, CDCl₃) δ ppm 23.7 (CH₃), 24.8 (CH₂), 24.9 (CH₂), 25.1 (CH₂), 26.6 (CH₂), 28.6 (CH₂), 28.9 (CH₂), 29.33 (CH₂), 30.8 (CH₃), 35.9 (CH₂), 47.1 (CH₂), 53.6 (CH), 54.4 (CH), 57.9 (CH), 58.9 (C), 126.9 (CH), 128.8 (2×CH), 129.2 (2×CH), 137.2 (C), 172.0 (C), 173.0 (C), 174.4 (C),175.8 (C), 196.0 (C) ; Masse (ESI+) *mlz* (%) 539 (100) [*M*+Na]⁺, 517 (10) ; SMHR (ESI+) : *m*/*z* calculée pour C₂₆H₃₆N₄O₅NaS 539.2304, trouvée 539.2302.

### Procédure générale pour obtenir (IV') à partir de (VI) en « un pot », exemple de (IV'f)

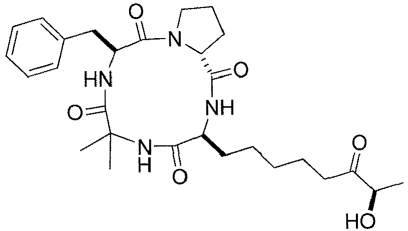

Le cyclotétrapeptide (VI) (31 mg, 0.07 mmol) et le carbonyl xanthate (IIIf) (45 mg, 0.14 mmol) dans du 1,2-dichloroéthane (0.2 mL) sont chauffés à reflux pendant 30 min sous argon. 3 mg de dilauroyl peroxide (DLP) sont ajoutés tous les 2 h (5 à 7 fois en tout). Au bout de 18 h à reflux (réaction suivie par CCM), le solvant est évaporé et remplacé par de l'isopropanol (1.5 mL). La solution est portée 30 min à reflux sous argon, puis du DLP (28 mg, 0.07 mmol) est ajouté. La solution est chauffée à reflux pendant 1 h, puis du DLP est à nouveau ajouté (14 mg, 0.035 mmol). Le mélange est chauffé encore 3 h. Après évaporation, le produit est directement purifié par chromatographie (gel de silice, 25% EtOAc/cyclohexane) pour donner le produit (IV'f) (22 mg, 48%).

### Préparation du (3S,9S,14aR)-9-benzyl-6,6-diméthyl-3-(7,7,7-trifluoro-6-oxoheptyl) décahydropyrrolo[1,2-a][1,4,7,10]tétraazacyclododécine-1,4,7,10-tétraone (IV'b)

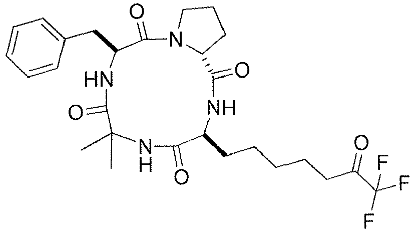

Le produit (IV'b) est obtenu après purification par flash chromatographie (35% EtOAc/cyclohexane), Rdt% = 49% ; R_{f} = 0.08 (40% EtOAc/cyclohexane) ; [α]^{D} = -71 (c = 0.44, CHCl₃) ; RMN ¹H (400 MHz, CDCl₃) δ ppm 1.25-1.36 (m, 7H), 1.56-1.72 (m, 4H), 1.77 (m, 1 H), 1.77 (s, 3H), 2.18 (m, 1H), 2.33 (m, 1H), 2.71 (t, *J* = 7.2 Hz, 2H), 2.95 (dd, *J* = 13.5, 5.7 Hz, 1H), 3.22 (m, 1H), 3.26 (dd, *J* = 13.5, 10.1 Hz, 1 H), 3.87 (m, 1 H), 4.19 (m, 1 H), 4.66 (bd, *J* = 7.8 Hz, 1H), 5.17 (ddd, *J* = 10.1, 10.1, 5.7 Hz, 1H), 5.92 (s, NH), 7.12 (d, *J* = 10.2 Hz, NH), 7.19-7.31 (m, 5H), 7.49 (d, *J* = 10.2 Hz, NH) ; IR vₘₐₓ (thin film, CH₂Cl₂) 3307, 2925, 1763, 1683, 1666, 1631, 1529, 1435, 1206, 1174 cm⁻¹; Masse (ESI+) *mlz* (%) 575 (100) [*M*+Na]⁺, 437 (12) ; SMHR (ESI+) : *m*/*z* calculée pour C₂₇H₃₅N₄O₅F₃Na 575.2457, trouvée 575.2442.

### Préparation du (3S,9S,14aR)-9-benzyl-6,6-dimethyl-3-(6-oxooctyl) decahydropyrrolo[1,2-a][1,4,7,10]tetraazacyclododecine-1,4,7,10-tetraone (IV'd)

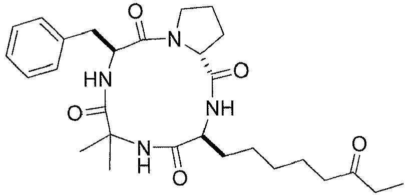

Le produit (IV'd) est obtenu après purification par flash chromatographie (30% EtOAc/cyclohexane), Rdt% = 50% ; R*_{f}* = 0.1 (40% EtOAc/cyclohexane) ; [α]^{D} = -65 (c = 0.47, CHCl₃) ; IR vₘₐₓ (film) 2923, 1716, 1683, 1540, 1522, 1457, 1177 cm⁻¹ ; RMN ¹H (400 MHz, CDCl₃) δ ppm 1.05 (t, *J* = 7.3 Hz, 3H), 1.25-1.34 (m, 4H), 1.34 (s, 3H), 1.51-1.70 (m, 4H), 1.77 (m, 1H), 1.77 (s, 3H), 2.18 (m, 1H), 2.32 (m, 1H), 2.39 (t, J *=* 7.3 Hz, 2H), 2.41 (q, *J* = 7.3 Hz, 2H), 2.95 (dd, *J* = 13.5, 5.7 Hz, 1H), 3.22 (m, 1H), 3.26 (dd, *J* = 13.5, 9.9 Hz, 1 H), 3.86 (m, 1 H), 4.18 (m, 1 H), 4.66 (bd, *J* = 8.0 Hz, 1 H), 5.16 (ddd, *J =* 10.1, 10.1, 5.8 Hz, 1H), 5.89 (s, NH), 7.08 (d, *J* = 10.2 Hz, NH), 7.18-7.31 (m, 5H), 7.51 (d, *J* = 10.2 Hz, NH) ; RMN ¹³C (100 MHz, CDCl₃) δ ppm 8.1 (CH₃), 23.8 (CH₃), 25.0 (CH₂), 25.2 (CH₂), 25.6 (CH₂), 26.7 (CH₃), 29.0 (CH₂), 29.9 (CH₂), 36.0 (CH₂), 36.1 (CH₂), 42.4 (CH₂), 47.2 (CH₂), 53.6 (CH), 54.5 (CH), 58.0 (CH), 59.0 (C), 126.9 (CH), 128.8 (2xCH), 129.2 (2xCH), 137.2 (C), 172.0 (C), 173.0 (C), 174.6 (C), 175.9 (C), 211.8 (C) ; Masse (ESI+) *m*/*z (%)* 535 (100) [*M*+Na]⁺, 437 (33).

### Préparation de l'éthyl 8-((3S,9S,14aR)-9-benzyl-6,6-diméthyl-1,4,7,10-tétraoxotétradécahydropyrrolo[1,2-a][1,4,7,10]tétraazacyclododécin-3-yl)-3-oxooctanoate (IV'e)

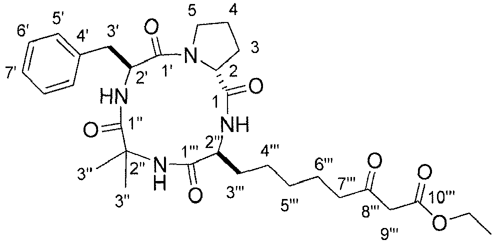

Le produit (IV'e) est obtenu après purification par flash chromatographie (35% EtOAc/cyclohexane), Rdt% = 52% ; R*_{f}* = 0.05 (40% EtOAc/cyclohexane) ; [α]^{D} = -71 (c = 0.68, CHCl₃) ; IR vₘₐₓ (film) 3305, 2928, 1741, 1714, 1681, 1666, 1629, 1529, 1434, 1315, 1232, 1178, 1030 cm⁻¹ ; RMN ¹H (400 MHz, CDCl₃) δ ppm 1.28 (t, *J* = 7.1Hz, 3H), 1.25-1.34 (m, 4H), 1.34 (s, 3H), 1.54-1.67 (m, 4H), 1.77 (m, 1H), 1.77 (s, 3H), 2.17 (m, 1H), 2.32 (m, 1H), 2.53 (t, *J* = 7.3 Hz, 2H), 2.95 (dd, *J* = 13.5, 5.7 Hz, 1H), 3.23 (m, 1H), 3.26 (dd, *J* = 13.4, 10.0 Hz, 1 H), 3.42 (s, 2H), 3.86 (m, 1 H), 4.17 (m, 1H), 4.20 (q, *J* = 7.1 Hz, 2H), 4.66 (bd, *J* = 7.8 Hz, 1H), 5.16 (ddd, *J* = 10.1, 10.1, 5.8 Hz, 1 H), 5.92 (s, NH), 7.09 (d, *J* = 10.2 Hz, NH), 7.19-7.32 (m, 5H), 7.51 (d, *J* = 10.1 Hz, NH) ; RMN ¹³C (100 MHz, CDCl₃) δ ppm 14.3 (CH₃), 23.3 (CH₂), 23.8 (CH₃), 25.0 (CH₂), 25.2 (CH₂), 25.5 (CH₂), 26.7 (CH₃), 28.8 (CH₂), 28.9 (CH₂), 36.0 (CH₂), 43.0 (CH₂), 47.2 (CH₂), 49.5 (CH₂), 53.6 (CH), 54.5 (CH), 58.0 (CH), 59.0 (C), 61.6 (CH₂), 126.9 (CH), 128.8 (2×CH), 129.2 (2×CH), 137.2 (C), 167.4 (C), 172.0 (C), 173.0 (C), 174.5 (C), 175.8 (C), 202.9 (C) ; Masse (ESI+) *m*/*z* (%) 593 (100) [*M*+Na]⁺, 521 (20) ; SMHR (ESI+) : *m*/*z* calculée pour C₃₀H₄₂N₄O₇Na 593.2951, trouvée 593.2942.

### 4. Procédé de préparation des peptides cycliques de formule (IV'f) et (IV'g)

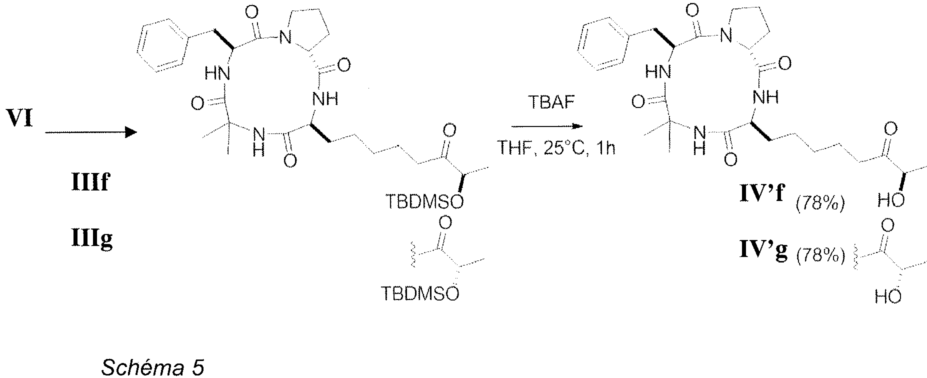

Selon un mode de réalisation de l'invention, on fait réagir le composé intermédiaire de formule (VII) avec le carbonyle xanthate de formule (IIIf) (voir *schéma* 2) et dans une seconde étape, on procède à la déprotection du composé obtenu. On obtient ainsi le composé (IV'f). Selon un autre mode de réalisation de l'invention, on fait réagir le composé intermédiaire de formule (VII) avec le carbonyle xanthate de formule (IIIg) (voir *schéma* 2) de manière à obtenir le composé (IV'g).

La déprotection des groupements silylés (TMDMS) est réalisée avec l'aide du fluorure de tétrabutyl ammonium (TBAF).

### (3S,9S, 14aR)-9-benzyl-3-((R)-7-hydroxy-6-oxooctyl)-6,6-diméthyldécahydropyrrolo[1,2-a][1,4,7,10]tétraazacyclododécine-1,4,7,10-tétraone (IV'f)

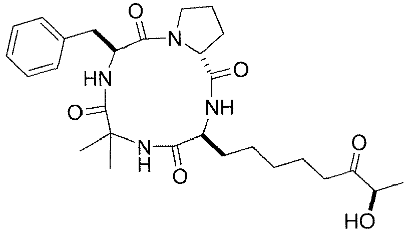

R*_{f}* = 0.16 (2% MeOH/CH₂Cl₂) ; RMN ¹H (400 MHz, CDCl₃) δ ppm 1.26-1.35 (m, 4H), 1.34 (s, 3H), 1.38 (d, *J =* 7.1 Hz, 3H), 1.60-1.69 (m, 4H), 1.77 (m, 1H), 1.77 (s, 3H), 2.18 (m, 1 H), 2.33 (m, 1H), 2.40-2.57 (m, 2H), 2.95 (dd, *J*= 13.5, 5.7 Hz, 1H), 3.21 (m, 1H), 3.27 (dd, *J* = 13.5, 10.1 Hz, 1H), 3.86 (m, 1H), 4.19 (m, 1H), 4.24 (q, *J* = 7.1 Hz, 1H), 4.66 (bd, *J* = 7.7 Hz, 1H), 5.17 (ddd, *J* = 10.1, 10.1, 5.8 Hz, 1H), 6.00 (s, NH), 7.13 (d, *J* = 10.2 Hz, NH), 7.18-7.30 (m, 5H), 7.51 (d, *J* = 10.2 Hz, NH) ; RMN ¹³C (100 MHz, CDCl₃) δ ppm 20.1 (CH₃), 23.5 (CH₂), 23.8 (CH₃), 25.0 (CH₂), 25.2 (CH₂), 25.4 (CH₂), 26.7 (CH₃), 28.9 (CH₂), 29.0 (CH₂), 36.0 (CH₂), 37.5 (CH₂), 47.2 (CH₂), 53.7 (CH), 54.5 (CH), 58.0 (CH), 59.0 (C), 72.8 (CH), 126.9 (CH), 128.8 (2×CH), 129.2 (2×CH), 137.2 (C), 172.1 (C), 173.0 (C), 174.5 (C), 175.8 (C), 212.6 (C); Masse (ESI+) *m*/*z* (%) 551 (100) [*M*+Na]⁺, 529 (10); SMHR (ESI+) : *mlz* calculée pour C₂₈H₄₀N₄O₆Na 551.2846, trouvée 551.2847.

### (3S,9S,14aR)-9-benzyl-3-((S)-7-hydroxy-6-oxooctyl)-6,6-diméthyldécahydropyrrolo[1,2-a][1,4,7,10]tétraazacyclododécine-1,4,7,10-tétraone (IV'g)

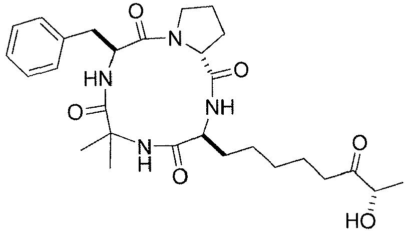

R*_{f}* = 0.09 (60% EtOAc/cyclohexane) ; ¹H NMR (400 MHz, CDCl₃) δ ppm 1.25-1.35 (m, 4H), 1.34 (s, 3H), 1.38 (d, *J* = 7.1 Hz, 3H), 1.62-1.69 (m, 4H), 1.77 (m, 1H), 1.77 (s, 3H), 2.17 (m, 1 H), 2.32 (m, 1 H), 2.38-2.58 (m, 2H), 2.95 (dd, *J* = 13.5, 5.7 Hz, 1 H), 3.22 (m, 1 H), 3.26 (dd, *J* = 13.5, 10.1 Hz, 1 H), 3.86 (m, 1 H), 4.19 (m, 1 H), 4.22 (q, *J =* 7.1 Hz, 1H), 4.67 (bd, *J* = 7.7 Hz, 1 H), 5.16 (ddd, *J* = 10.1, 10.1, 5.8 Hz, 1 H), 6.12 (bs, NH), 7.17 (d, *J* = 10.2 Hz, NH), 7.20-7.30 (m, 5H), 7.54 (d, *J* = 10.2 Hz, NH) ; RMN ¹³C (100 MHz, CDCl₃) δ ppm 20.1 (CH₃), 23.5 (CH₂), 23.8 (CH₃), 25.0 (CH₂), 25.2 (CH₂), 25.5 (CH₂), 26.7 (CH₃), 28.9 (CH₂), 29.0 (CH₂), 36.0 (CH₂), 37.5 (CH₂), 47.2 (CH₂), 53.7 (CH), 54.5 (CH), 58.0 (CH), 59.0 (C), 72.8 (CH), 126.9 (CH), 128.8 (2×CH), 129.2 (2xCH), 137.2 (C), 172.1 (C), 173.0 (C), 174.5 (C), 175.8 (C), 212.6 (C); Masse (ESI+) *mlz* (%) 551 (45) [*M*+Na]⁺, 242 (100); SMHR (ESI+) : *mlz* calculée pour C₂₈H₄₀N₄O₆Na 551.2846, trouvée 551.2847.

### 5. Procédé de préparation d'autres peptides cycliques selon l'invention

### Préparation des peptides cycliques (IV'j) et (IV'k)

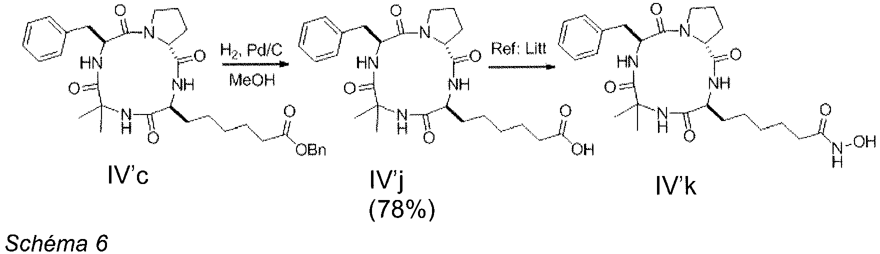

L'acide (IV'j) est obtenu par hydrogénation catalytique. L'acide hydroxamique (IV'k) est obtenu à partir de (IV'j).

### Préparation du peptide cyclique (3S,9S,14aR)-9-benzyl-3-(7-bromo-6-oxoheptyl)-6,6-diméthyldécahydropyrrolo[1,2-a][1,4,7,10]tétraazacyclododécine-1,4,7,10-tétraone (IVₕ)

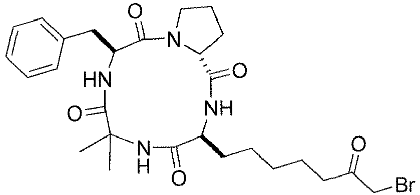

On ajoute du LiBr (23 mg, 0.27 mmol) au composé (IV'a) (7.1 mg, 0.013 mmol) dissous dans l'acétone (1 mL) sous argon. La solution est agitée à l'abri de la lumière pendant 5 jours. L'acétone est évaporée et de l'EtOAc est ajouté. La phase organique est lavée avec de l'eau, séchée sur MgSO₄, puis évaporée. Après flash chromatographie (gel de silice, 2% MeOH/CH₂Cl₂), le produit (IVh) (5.8 mg, 75%) est isolé sous forme de laque incolore.
R*_{f}* = 0.07 (40% EtOAc/cyclohexane) ; RMN ¹H (400 MHz, CDCl₃) δ ppm 1.27-1.34 (m, 4H), 1.34 (s, 3H), 1.62 (m, 2H), 1.70-1.83 (m, 7H), 2.17 (m, 1 H), 2.32 (m, 1 H), 2.65 (t, *J* = 7.3 Hz, 2H), 2.95 (dd, *J* = 13.5, 5.7 Hz, 1H), 3.24 (m, 1H), 3.26 (dd, *J* = 13.5, 10.0 Hz, 1 H), 3.86 (m, 1H), 3.88 (s, 2H), 4.18 (ddd, *J =* 10.2, 7.6, 7.6 Hz, 1H), 4.65 (bd, *J =* 7.8 Hz, 1H), 5.16 (ddd, *J* = 10.1, 10.0, 5.7 Hz, 1H), 5.93 (s, NH), 7.11 (bd, *J* = 10.1 Hz, NH), 7.19-7.31 (m, 5H), 7.51 (bd, *J* = 10.1 Hz, NH) ; RMN ¹³C (100 MHz, CDCl₃) δ ppm 23.7 (CH₃, CH₂), 25.0 (CH₂), 25.2 (CH₂), 25.5 (CH₂), 26.7 (CH₃), 28.8 (CH₂), 28.9 (CH₂), 34.5 (CH₂), 36.0 (CH₃), 39.8 (CH₂), 47.2 (CH₂), 53.6 (CH), 54.5 (CH), 58.0 (CH), 59.0 (C), 126.9 (CH), 128.8 (2×CH), 129.3 (2×CH), 137.2 (C), 172.1 (C), 173.0 (C), 174.5 (C),175.8 (C), 202.2 (C) ; Masse (ESI+) *m*/*z* (%) 602 (30) [*M*+Na]⁺, 601 (100) [*M*+Na]⁺, 600 (30) [*M*+Na]⁺, 599 (100) [*M*+Na]⁺, 555 (30).

### Préparation du peptide cyclique S-7-((3S,9S,14aR)-9-benzyl-6,6-diméthyl-1,4,7,10-tétraoxotétradécahydropyrrolo[1,2-a][1,4,7,10]tétraazacyclododécin-3-yl)-2-oxoheptyl O-éthyl carbonodithioate (IVi)

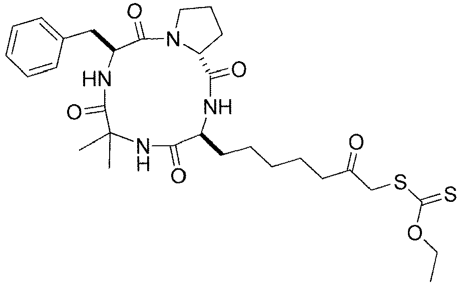

L'éthyl xanthate de potassium (4.2 mg, 0.026 mmol) dans l'acétone (0.2 mL) est injecté à une solution de composé (IV'a) (7.9 mg, 0.0147 mmol) dans l'acétone (0.4 mL) à 0°C. La réaction est agitée à température ambiante pendant 5 h. Le solvant est évaporé et le résidu est directement purifié par flash chromatographie pour donner le composé (IVi) (5.5 mg, 60%).
R*_{f}* = 0.07 (40% EtOAc/cyclohexane) ; RMN ¹H (400 MHz, CDCl₃) δ ppm 1.28-1.34 (m, 4H), 1.34 (s, 3H), 1.42 (t, *J* = 7.1 Hz, 3H), 1.59-1.68 (m, 4H), 1.77 (m, 1H), 1.77 (s, 3H), 2.17 (m, 1H), 2.32 (m, 1H), 2.59 (t, *J* = 7.3 Hz, 2H), 2.95 (dd, *J* = 13.5, 5.7 Hz, 1 H), 3.23 (m, 1 H), 3.26 (dd, *J* = 13.5, 9.9 Hz, 1H), 3.86 (m, 1H), 4.17 (m, 1H), 4.65 (bd, *J* = 7.1 Hz, 2H), 6.67 (m, 1 H), 5.16 (ddd, *J =* 10.1, 10.1, 5.8 Hz, 1 H), 5.88 (s, NH), 7.09 (d, *J* = 10.2 Hz, NH), 7.18-7.29 (m, 5H), 7.50 (d, *J* = 10.2 Hz, NH) ; RMN ¹³C (100 MHz, CDCl₃) δ ppm 23.6 (CH₂), 23.8 (CH₃), 25.0 (CH₂), 25.3 (CH₂), 25.5 (CH₂), 26.7 (CH₃), 28.9 (CH₂), 29.0 (CH₂), 36.0 (CH₂), 41.9 (CH₂), 45.7 (CH₂), 47.2 (CH₂), 53.7 (CH), 54.5 (CH), 58.0 (CH), 59.0 (C), 71.1 (CH₂), 126.9 (CH), 128.8 (2×CH), 129.2 (2xCH), 137.2 (C), 172.0 (C), 173.0 (C), 174.5 (C), 175.8 (C), 203.4 (C), 213.1 (C); Masse (ESI+) *mlz* (%) 641 (100) [*M*+Na]⁺, 553 (85) ; SMHR (ESI+) : *mlz* calculée pour C₃₀H₄₂N₄O₆NaS₂ 641.2443, trouvée 641.2438.

### 6. Tests biologiques sur cellules

Les peptides cycliques synthétisés par un procédé selon l'invention, mais n'entrant pas dans la portée de l'invention en tant que tels, ont fait l'objet de tests *in vitro* sur fibroblastes humains infectés par *Toxoplasma gondii* (Toxo), sur fibroblastes humains (HFF) non infectés et sur cellules cancéreuses HeLa (cancer cervical) et MCF-7 (cancer du sein). Leurs efficacités respectives ont été testées à 90 nM comme test préliminaire.

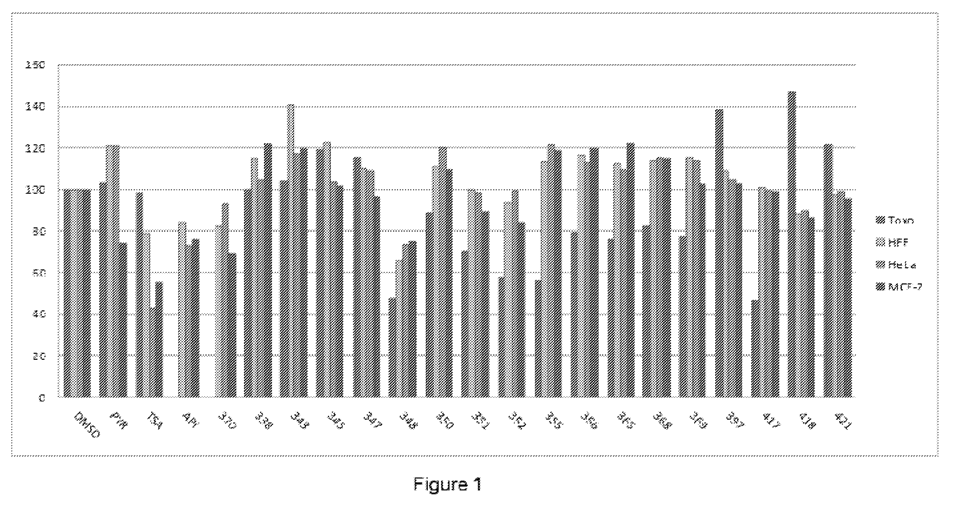

### Les résultats sont représentés à la figure 1.

Correspondance des molécules : API=Apicidine , 370=(IV'f), 338= (VI), 343= (IVc), 345= (IV'c), 347= (IV'j), 348= (IV'k), 350= (IVa), 351= (IV'a), 355= (IVg), 356= (IV'g), 357= (IV'g), 368= (IVf), 369= (IV'f), 397= (XVIIIa), 417= (IV'd), 418= (IV'b), 421= (IV'e).

Ayant montré des résultats préliminaires intéressants, l'efficacité du peptide cyclique (IV'_{f}) sur des fibroblastes humains infectés par *Toxoplasma gondii* a ensuite été comparée à celle d'autres molécules connus (apicidine et pyriméthamine notamment).

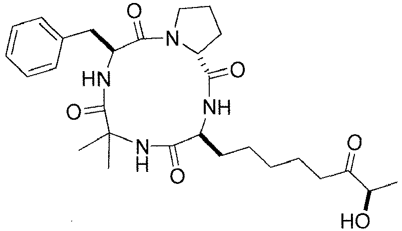

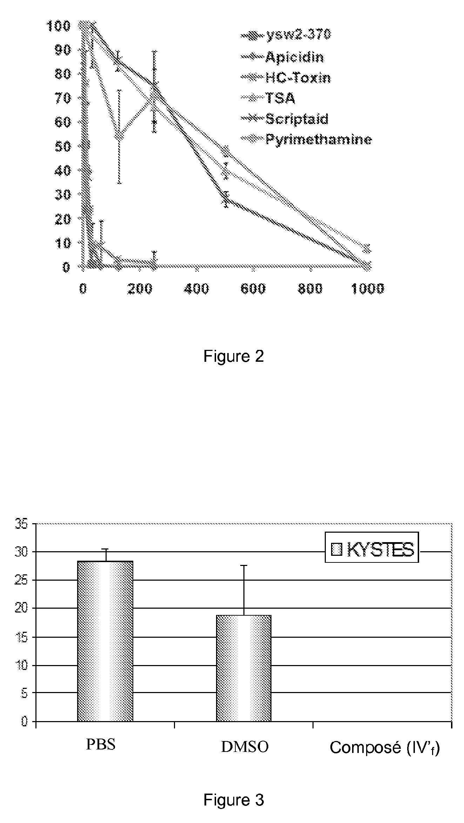

Comme représenté à la figure 2, le composé (IV'f) a montré une très bonne efficacité, de l'ordre du nanomolaire. Il a une activité proche de celle de l'apicidine et surtout une efficacité bien supérieure par rapport à la pyriméthamine qui est le composé utilisé actuellement dans le traitement de la toxoplasmose. Le tableau ci-dessous donne les valeurs numériques obtenues.

| composé (IV'_{f}) | Toxo | HFF | HeLa | MCF-7 |
|---|---|---|---|---|
| EC₅₀ (nM) | 28.6 | 647 | 474 | 533 |
| EC₉₀ (nM) | 79.7 | 4110 | 1012 | n.d. |

### 7. Tests biologiques sur cerveaux de souris

Le peptide cyclique (IV'f) a ensuite été testé sur des kystes (forme bradyzoïtes) de cerveaux de souris *ex vivo* à 200 nM (figure 3). Les kystes après traitement pendant 7 jours avec le peptide cyclique (IV'f) sont réinoculés de façon intra-péritoniale aux souris. Les souris ayant subi l'injection de kystes traités uniquement avec le tampon phosphate salin (PBS) ou le DMSO développent des kystes, alors que celles ayant subi une injection de kystes traités avec le peptide cyclique (IV'f) ne présentent aucun kyste.

## Revendications

1. Procédé de préparation, d'un peptide cyclique de formule (IV) : 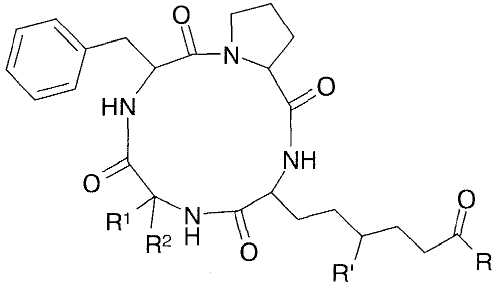 dans laquelle
R représente H ; OH; SH ; un groupe amine ; un groupe alkyle, haloalkyle ou hétéroalkyle contenant entre 1 et 10 atomes de carbone en chaîne linéaire ou ramifiée ; un groupe alcènyle ou alcynyle contenant entre 2 à 10 atomes de carbone en chaîne linéaire ou ramifiée ; un ou plusieurs groupes cycloalkyle, cycloacènyle ou cycloalcynyle contenant entre 3 à 10 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 10 atomes de carbone, les termes aryle et alkyle ayant les définitions ci-dessus ; un groupe alkoxy, thioalkyle, sulfonylalkyle, aminoalkyle contenant entre 1 et 10 atomes de carbone en chaîne linéaire ou ramifiée ; un groupe alkoxyalkyle, alkylthioalkyle, alkylsulfonylalkyle, alkylaminoalkyle contenant entre 1 et 30 atomes de carbone en chaîne linéaire ou ramifiée ; un ou plusieurs groupes hétérocyclique contenant entre 5 et 10 atomes de carbone par cycle ;
R' représente H ou un groupement xanthate de formule S-CS-O-R'" dans laquelle R'" représente H ; un groupe alkyle ou haloalkyle contenant entre 1 et 10 atomes de carbone en chaîne linéaire ou ramifiée ;
R¹ et R², identiques ou différents, représentent H ; OH ; SH ; un groupe amine ; un groupe alkyle, haloalkyle ou hétéroalkyle contenant entre 1 et 30 atomes de carbone en chaîne linéaire ou ramifiée ; un groupe alcènyle ou alcynyle contenant entre 2 à 30 atomes de carbone en chaîne linéaire ou ramifiée ; un ou plusieurs groupes cycloalkyle, cycloacènyle ou cycloalcynyle contenant entre 3 à 30 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 30 atomes de carbone, les termes aryle et alkyle ayant les définitions ci-dessus ; un groupe alkoxy, thioalkyle, sulfonylalkyle, aminoalkyle contenant entre 1 et 30 atomes de carbone en chaîne linéaire ou ramifiée ; un groupe alkoxyalkyle, alkylthioalkyle, alkylsulfonylalkyle, alkylaminoalkyle contenant entre 1 et 30 atomes de carbone en chaîne linéaire ou ramifiée ; un ou plusieurs groupes hétérocycliques contenant entre 5 et 10 atomes par cycle, saturés ou insaturés, comprenant au moins un hétéroatome choisi parmi N, O et S, le ou lesdits groupes hétérocycliques pouvant être substitués, et étant liés directement ou indirectement par un radical alkylène bivalent au cycle dudit peptide,
**caractérisé en ce que** l'on fait réagir un composé intermédiaire de formule (V) : 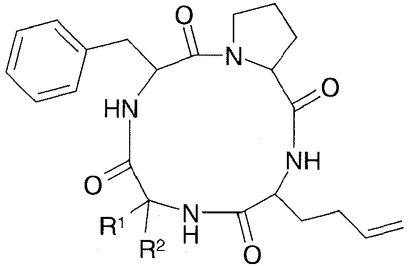 dans laquelle R¹ et R² ont la définition ci-dessus, avec un carbonyle xanthate de formule (III) : 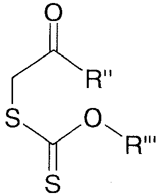 dans laquelle
R" représente H ; un groupe amine ; un groupe alkyle, haloalkyle ou hétéroalkyle contenant entre 1 et 10 atomes de carbone en chaîne linéaire ou ramifiée ; un groupe alcènyle ou alcynyle contenant entre 2 à 10 atomes de carbone en chaîne linéaire ou ramifiée ; un ou plusieurs groupes cycloalkyle, cycloacènyle ou cycloalcynyle contenant entre 3 à 10 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 10 atomes de carbone, les termes aryle et alkyle ayant les définitions ci-dessus ; un groupe alkoxy, thioalkyle, sulfonylalkyle, aminoalkyle contenant entre 1 et 10 atomes de carbone en chaîne linéaire ou ramifiée ; un groupe alkoxyalkyle, alkylthioalkyle, alkylsulfonylalkyle, alkylaminoalkyle contenant entre 1 et 10 atomes de carbone en chaîne linéaire ou ramifiée ; un ou plusieurs groupes hétérocyclique contenant entre 5 et 10 atomes de carbone par cycle ;
R'" représente H ; un groupe alkyle ou haloalkyle contenant entre 1 et 10 atomes de carbone en chaîne linéaire ou ramifiée ;
et **en ce que**, éventuellement dans une seconde étape, on procède à la déprotection du composé obtenu.

2. Procédé selon la revendication 1, dans lequel le groupement R du peptide cyclique de formule (IV) représente H ; OH, SH ; un groupe alkyle ou haloalkyle contenant entre 1 et 10 atomes de carbone en chaîne linéaire ou ramifiée ; un ou plusieurs groupes aryle ou hétéroaryle contenant entre 3 à 10 atomes de carbone par cycle ; un groupe alkoxy, thioalkyle, sulfonylalkyle, aminoalkyle contenant entre 1 et 10 atomes de carbone en chaîne linéaire ou ramifiée ; un ou plusieurs groupes hétérocycliques contenant entre 5 et 10 atomes de carbone par cycle.

3. Procédé selon la revendication 2, dans lequel le groupement R du peptide cyclique de formule (IV) est choisi parmi CH₂Cl, CH₂Br, CF₃, OH, O-CH₂-C₆H₆, NHOH, CH₂-S-CS-O-CH₂CH₃, CH₂CH₃, CO-CH₂CO₂-CH₂CH₃, CHOH-CH₃, CH(OTBDMS)CH₃.

4. Procédé selon l'une quelconque des revendications précédentes, dans lequel le groupement R' du peptide cyclique de formule (IV) représente H.

5. Procédé selon l'une quelconque des revendications précédentes, dans lequel le groupement R" du composé de formule (III) représente H ; un groupe alkyle ou haloalkyle contenant entre 1 et 10 atomes de carbone en chaîne linéaire ou ramifiée ; un ou plusieurs groupes aryle ou hétéroaryle contenant entre 3 à 10 atomes de carbone par cycle ; un groupe alkoxy, thioalkyle, sulfonylalkyle, aminoalkyle contenant entre 1 et 10 atomes de carbone en chaîne linéaire ou ramifiée ; un ou plusieurs groupes hétérocycliques contenant entre 5 et 10 atomes de carbone par cycle.

6. Procédé selon la revendication 5, dans lequel le groupement R" du composé de formule (III) est choisi parmi CH₂Cl, CH₂Br, CF₃, O-CH₂-C₆H₆, CH₂CH₃, COCH₂CO₂Et, CH(OTBDMS)CH₃.

7. Composé intermédiaire de synthèse de formule (V) : 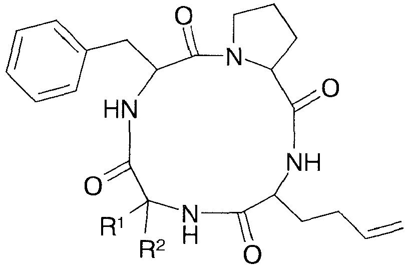 Dans laquelle R¹ et R² sont tels que définis à l'une quelconque des revendications 1 à 3.

8. Composé selon la revendication 7, **caractérisé en ce qu'**au moins l'un de R¹ et R² représente CH₂C≡CH, l'autre de R¹ et R² représentant H, le cas échéant.

9. Peptide cyclique, de formule (IV) : 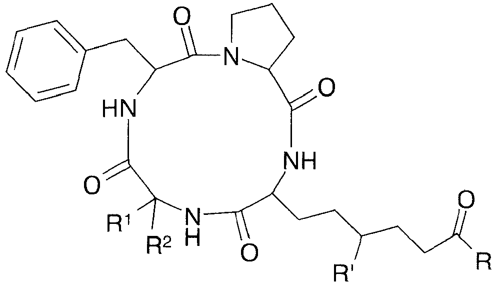 dans laquelle,
R représente H ; OH ; SH ; un groupe amine ; un groupe alkyle, haloalkyle ou hétéroalkyle contenant entre 1 et 10 atomes de carbone en chaîne linéaire ou ramifiée ; un groupe alcènyle ou alcynyle contenant entre 2 à 10 atomes de carbone en chaîne linéaire ou ramifiée ; un ou plusieurs groupes cycloalkyle, cycloacènyle ou cycloalcynyle contenant entre 3 à 10 atomes de carbone en chaîne linéaire ou ramifiée ; un ou plusieurs groupes aryle ou hétéroaryle contenant entre 3 à 10 atomes de carbone par cycle ; un groupe alkaryle ou aralkyle contenant entre 1 et 10 atomes de carbone, les termes aryle et alkyle ayant les définitions ci-dessus ; un groupe alkoxy, thioalkyle, sulfonylalkyle, aminoalkyle contenant entre 1 et 10 atomes de carbone en chaîne linéaire ou ramifiée ; un groupe alkoxyalkyle, alkylthioalkyle, alkylsulfonylalkyle, alkylaminoalkyle contenant entre 1 et 30 atomes de carbone en chaîne linéaire ou ramifiée ; un ou plusieurs groupes hétérocyclique contenant entre 5 et 10 atomes de carbone par cycle ;
R' représente H ou un groupement xanthate de formule S-CS-O-R'" dans laquelle R'" représente H ; un groupe alkyle ou haloalkyle contenant entre 1 et 10 atomes de carbone en chaîne linéaire ou ramifiée ;
au moins l'un de R¹ et R² représente CH₂C≡CH ; l'autre de R¹ et R² représentant H, le cas échéant.

10. Peptide cyclique selon la revendication 9, dans lequel R' représente H.

11. Peptide cyclique selon l'une quelconque des revendications 9 à 10, dans lequel R représente CHOH-CH₃.

12. Peptide selon l'une quelconque des revendications 9 à 11, pour son utilisation comme agent antiparasitaire.

13. Peptide selon la revendication 12, pour le traitement de la malaria, la toxoplasmose, la coccidiose, la cryptosporidiose ou la néosporose.

14. Peptide selon l'une quelconque des revendications 9 à 11, pour le traitement du cancer.

15. Utilisation d'un peptide selon l'une quelconque des revendications 9 à 11, pour traiter un organe *ex vivo* avant transplantation.

## Patentansprüche

1. Verfahren zur Herstellung eines cyclischen Peptids mit der folgenden Formel (IV): 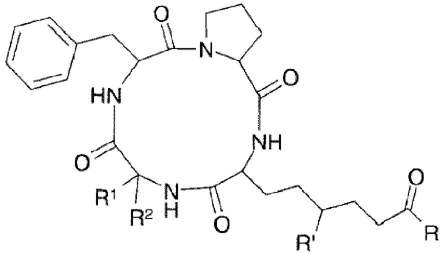 wobei
R H; OH; SH darstellt; eine Aminogruppe; eine Alkyl-, Haloalkyl- oder Heteroalkylgruppe, enthaltend zwischen 1 und 10 Kohlenstoffatome in linearer oder verzweigter Kette; eine Alcenyl- oder Alcynylgruppe, enthaltend zwischen 2 bis 10 Kohlenstoffatome in linearer oder verzweigter Kette; eine oder mehrere Cycloalkyl-, Cycloacenyl- oder Cycloalcynylgruppen, enthaltend zwischen 3 bis 10 Kohlenstoffatome in linearer oder verzweigter Kette; eine oder mehrere Aryl- oder Heteroarylgruppen, enthaltend zwischen 3 bis 10 Kohlenstoffatome pro Zyklus; eine Alkaryl oder Aralkylgruppe, enthaltend zwischen 1 und 10 Kohlenstoffatome, wobei die Ausdrücke Aryl und Alkyl die oben angegebenen Definitionen aufweisen; eine Alkoxy-, Thioalkyl-, Sulfonylalkyl-, Aminoalkylgruppe, enthaltend zwischen 1 und 10 Kohlenstoffatome in linearer oder verzweigter Kette; eine Alkoxyalkyl-, Alkylthioalkyl-, Alkylsulfonylalkyl-, Alkylaminoalkylguppe, enthaltend zwischen 1 und 30 Kohlenstoffatome in linearer oder verzweigter Kette; eine oder mehrere heterocyclische Gruppen, enthaltend zwischen 5 und 10 Kohlenstoffatome pro Zyklus;
R' H oder eine Xanthat-Gruppierung mit der Formel S-CS-O-R"' darstellt, bei der R"' H darstellt; eine Alkyl- oder Haloalkylgruppe, enthaltend zwischen 1 und 10 Kohlenstoffatome in linearer oder verzweigter Kette;
R¹ und R², identisch oder verschieden, H; OH; SH darstellen; eine Aminogruppe; eine Alkyl-, Haloalkyl- oder Heteroalkylgruppe, enthaltend zwischen 1 und 30 Kohlenstoffatome in linearer oder verzweigter Kette; eine Alcenyl- oder Alcynylgruppe, enthaltend zwischen 2 bis 30 Kohlenstoffatome in linearer oder verzweigter Kette; eine oder mehrere Cycloalkyl-, Cycloacenyl- oder Cycloalcynylgruppen, enthaltend zwischen 3 bis 30 Kohlenstoffatome in linearer oder verzweigter Kette; eine oder mehrere Aryl- oder Heteroarylgruppen, enthaltend zwischen 3 bis 10 Kohlenstoffatome pro Zyklus; eine Alkaryl oder Aralkylgruppe, enthaltend zwischen 1 und 30 Kohlenstoffatome, wobei die Ausdrücke Aryl und Alkyl die oben angegebenen Definitionen aufweisen; eine Akoxy-, Thioalkyl-, Sulfonylalkyl-, Aminoalkylgruppe, enthaltend zwischen 1 und 30 Kohlenstoffatome in linearer oder verzweigter Kette; eine Alkoxyalkyl-, Alkylthioalkyl-, Alkylsulfonylalkyl-, Alkylaminoalkylguppe, enthaltend zwischen 1 und 30 Kohlenstoffatome in linearer oder verzweigter Kette; eine oder mehrere heterocyclische Gruppen, enthaltend zwischen 5 und 10 Kohlenstoffatome pro Zyklus, gesättigt oder ungesättigt, umfassend mindestens ein Heteroatom, ausgewählt aus N, O und S, wobei die heterocyclische(n) Gruppe(n) substituiert sein kann/können und direkt oder indirekt durch ein bivalentes Alkylenradikal mit dem Zyklus des Peptids verbunden ist/sind,
**dadurch gekennzeichnet, dass** eine Zwischenverbindung mit der Folgenden Formel (V) zum Reagieren gebracht wird: 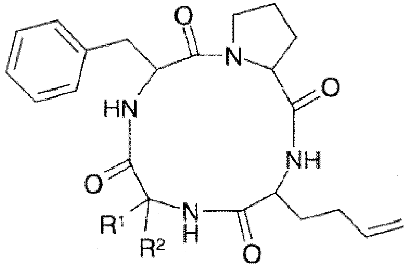 wobei R¹ und R² die unten angegebene Definition aufweisen, mit einem Carbonylxanthat mit der folgenden Formel (III): 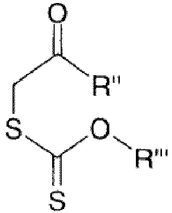 wobei
R" H darstellt; eine Aminogruppe; eine Alkyl, Haloalkyl- oder Heteroalkylgruppe, enthaltend zwischen 1 und 10 Kohlenstoffatome in linearer oder verzweigter Kette; eine Alcenyl- oder Alcynylgruppe, enthaltend zwischen 2 bis 10 Kohlenstoffatome in linearer oder verzweigter Kette; eine oder mehrere Cycloalkyl-, Cycloacenyl- oder Cycloalcynylgruppen, enthaltend zwischen 3 bis 10 Kohlenstoffatome in linearer oder verzweigter Kette; eine oder mehrere Aryl- oder Heteroarylgruppen, enthaltend zwischen 3 bis 10 Kohlenstoffatome pro Zyklus; eine Alkaryl oder Aralkylgruppe, enthaltend zwischen 1 und 10 Kohlenstoffatome, wobei die Ausdrücke Aryl und Alkyl die unten angegebenen Definitionen aufweisen; eine Alkoxy-, Thioalkyl-, Sulfonylalkyl-, Aminoalkylgruppe, enthaltend zwischen 1 und 10 Kohlenstoffatome in linearer oder verzweigter Kette; eine Alkoxyalkyl-, Alkylthioalkyl-, Alkylsulfonylalkyl-, Alkylaminoalkylguppe, enthaltend zwischen 1 und 10 Kohlenstoffatome in linearer oder verzweigter Kette; eine oder mehrere heterocyclische Gruppen, enthaltend zwischen 5 und 10 Kohlenstoffatome pro Zyklus;
R"' H darstellt; eine Alkyl- oder Haloalkylgruppe, enthaltend zwischen 1 und 10 Kohlenstoffatome in linearer oder verzweigter Kette;
und dadurch, dass, eventuell in einem zweiten Schritt, die Aufhebung des Schutzes der erhaltenen Verbindung vorgenommen wird.

2. Verfahren nach Anspruch 1, wobei die Gruppierung R des cyclischen Peptids mit der Formel (IV) H; OH; SH darstellt; eine Alkyl- oder Haloalkylgruppe, enthaltend zwischen 1 und 10 Kohlenstoffatome in linearer oder verzweigter Kette; eine oder mehrere Aryl- oder Heteroarylgruppen, enthaltend zwischen 3 und 10 Kohlenstoffatome pro Zyklus; eine Akoxy-, Thioalkyl-, Sulfonylalkyl-, Aminoalkylgruppe, enthaltend zwischen 1 und 10 Kohlenstoffatome in linearer oder verzweigter Kette; eine oder mehrere heterocyclische Gruppen, enthaltend zwischen 5 und 10 Kohlenstoffatome pro Zyklus.

3. Verfahren nach Anspruch 2, wobei die Gruppierung R des cyclischen Peptids mit der Formel (IV) ausgewählt ist aus CH₂Cl, CH₂Br, CF₃, OH, O-CH₂-C₆H₆, NHOH, CH₂-S-CS-O-CH₂CH₃, CH₂CH₃, CO-CH₂CO₂-CH₂CH₃, CHOH-CH₃, CH(OTBDMS)CH₃.

4. Verfahren nach einem der vorhergehenden Ansprüche, wobei die Gruppierung R' des cyclischen Peptids mit der Formel (IV) H darstellt.

5. Verfahren nach einem der vorhergehenden Ansprüche, wobei die Gruppierung R" der Verbindung mit der Formel (III) H darstellt; eine Alkyl- oder Haloalkylgruppe, enthaltend zwischen 1 und 10 Kohlenstoffatome in linearer oder verzweigter Kette; eine oder mehrere Aryl- oder Heteroarylgruppen, enthaltend zwischen 3 bis 10 Kohlenstoffatome pro Zyklus; eine Alkoxy-, Thioalkyl-, Sulfonylalkyl-, Aminoalkylgruppe, enthaltend zwischen 1 und 10 Kohlenstoffatome in linearer oder verzweigter Kette; eine oder mehrere heterocyclische Gruppen, enthaltend zwischen 5 und 10 Kohlenstoffatome pro Zyklus.

6. Verfahren nach Anspruch 5, wobei die Gruppierung R" der Verbindung mit der Formel (III) ausgewählt ist aus CH₂Cl, CH₂Br, CF₃, O-CH₂C₆H₆, CH₂CH₃, COCH₂CO₂Et, CH(OTBDMS)CH₃.

7. Synthesezwischenverbindung mit der folgenden Formel (V): 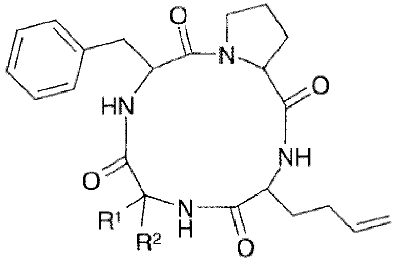 wobei der R¹ und R² wie in einem der Ansprüche 1 bis 3 definiert sind.

8. Verbindung nach Anspruch 7, **dadurch gekennzeichnet, dass** mindestens eines von R¹ und R² CH2C=CH darstellt, das andere von R¹ und R² gegebenenfalls H darstellt.

9. Cyclisches Peptid mit der folgenden Formel (IV): 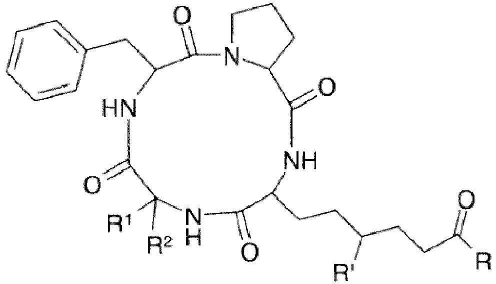 wobei
R H; OH; SH darstellt; eine Aminogruppe; eine Alkyl-, Haloalkyl- oder Heteroalkylgruppe, enthaltend zwischen 1 und 10 Kohlenstoffatome in linearer oder verzweigter Kette; eine Alcenyl- oder Alcynylgruppe, enthaltend zwischen 2 bis 10 Kohlenstoffatome in linearer oder verzweigter Kette; eine oder mehrere Cycloalkyl-, Cycloacenyl- oder Cycloalcynylgruppen, enthaltend zwischen 3 bis 10 Kohlenstoffatome in linearer oder verzweigter Kette; eine oder mehrere Aryl- oder Heteroarylgruppen, enthaltend zwischen 3 bis 10 Kohlenstoffatome pro Zyklus; eine Alkaryl oder Aralkylgruppe, enthaltend zwischen 1 und 10 Kohlenstoffatome, wobei die Ausdrücke Aryl und Alkyl die unten angegebenen Definitionen aufweisen; eine Alkoxy-, Thioalkyl-, Sulfonylalkyl-, Aminoalkylgruppe, enthaltend zwischen 1 und 10 Kohlenstoffatome in linearer oder verzweigter Kette; eine Alkoxyalkyl-, Alkylthioalkyl-, Alkylsulfonylalkyl-, Alkylaminoalkylguppe, enthaltend zwischen 1 und 30 Kohlenstoffatome in linearer oder verzweigter Kette; eine oder mehrere heterocyclische Gruppen, enthaltend zwischen 5 und 10 Kohlenstoffatome pro Zyklus;
R' H oder eine Xanthat-Gruppierung mit der Formel S-CS-O-R"' darstellt, bei der R"' H darstellt; eine Alkyl- oder Haloalkylgruppe, enthaltend zwischen 1 und 10 Kohlenstoffatome in linearer oder verzweigter Kette;
mindestens eines von R¹ und R² CH2=CH darstellt, das andere von R¹ und R² gegebenenfalls H darstellt.

10. Cyclisches Peptid nach Anspruch 9, wobei R' H darstellt.

11. Cyclisches Peptid nach einem der Ansprüche 9 bis 10, wobei R CHOH-CH₃ darstellt.

12. Peptid nach einem der Ansprüche 9 bis 11 für seine Verwendung als Schädlingsbekämpfungsmittel.

13. Peptid nach Anspruch 12 für die Behandlung von Malaria, Toxoplasmose, Kokzidiose, Kryptosporidiose oder Neosporose.

14. Peptid nach einem der Ansprüche 9 bis 11 für die Behandlung von Krebs.

15. Verwendung eines Peptids nach beliebigen der Ansprüche 9 bis 11 für die Behandlung eines Organs *ex vivo* vor der Transplantation.

## Claims

1. A preparation method of a cyclic peptide of the formula (IV): 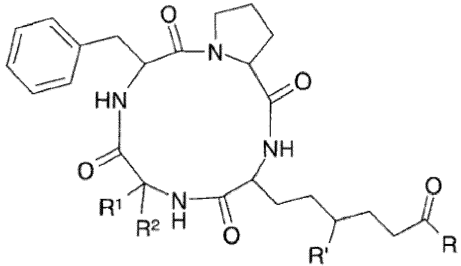 wherein
R represents H; OH; SH; an amino group; an alkyl, haloalkyl or heteroalkyl group containing from 1 to 10 carbon atom(s) in linear or branched chain; an alkenyl or alkynyl group containing from 2 to 10 carbon atoms in linear or branched chain; one or several cycloalkyl, cycloalkenyl or cycloalkynyl group(s) containing from 3 to 10 carbon atoms in linear or branched chain; one or several aryl or heteroaryl group(s) containing from 3 to 10 carbon atoms per ring; an alkaryl or aralkyl group containing from 1 to 10 carbon atom(s), the aryl and alkyl words having the above definitions; an alkoxy, thioalkyl, sulfonylalkyl, aminoalkyl group containing from 1 to 10 carbon atom(s) in linear or branched chain; an alkoxyalkyl, alkylthioalkyl, alkylsulfonylalkyl, alkylaminoalkyl group containing from 1 to 30 carbon atom(s) in linear or branched chain; one or several heterocyclic group(s) containing from 5 to 10 carbon atoms per ring;
R' represents H or a xanthate group of the formula S-CS-O-R'" wherein R"' represents H; an alkyl or haloalkyl group containing from 1 to 10 carbon atom(s) in linear or branched chain;
R¹ and R², identical or different, represent H; OH; SH; an amino group; an alkyl, haloalkyl or heteroalkyl group containing from 1 to 30 carbon atom(s) in linear or branched chain; an alkenyl or alkynyl group containing from 2 to 30 carbon atoms in linear or branched chain; one or several cycloalkyl, cycloalkenyl or cycloalkynyl group(s) containing from 3 to 30 carbon atoms in linear or branched chain; one or several aryl or heteroaryl group(s) containing from 3 to 10 carbon atoms per ring; an alkaryl or aralkyl group containing from 1 and 30 carbon atoms, the words aryl and alkyl having the definitions above; an alkoxy, thioalkyl, sulfonylalkyl, aminoalkyl group containing from 1 to 30 carbon atom(s) in linear or branched chain; an alkoxyalkyl, alkylthioalkyl, alkylsutfonylalkyl, alkylaminoalkyl group containing from 1 to 30 carbon atoms in linear or branched chain; one or several heterocyclic group(s) containing from 5 to 10 atoms per ring, saturated or unsaturated, comprising at least one heteroatom selected from N, O and S, said heterocyclic group(s) may be substituted, and is/are directly or indirectly linked, through a bivalent alkylene radical, to the cycle of said peptide,
**characterized in that** an intermediate compound of the formula (V) is reacted: 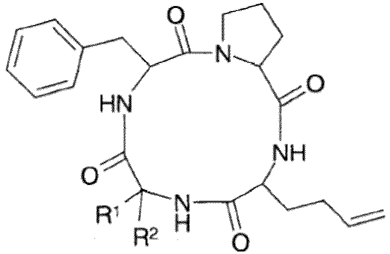 wherein R¹ and R² have the above definition,
with a xanthate carbonyl of the formula (III): 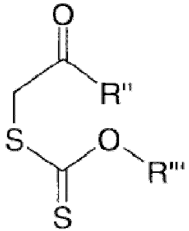 wherein
R" represents H; an amino group; an alkyl, haloalkyl or heteroalkyl group containing from 1 and 10 carbon atoms in linear or branched chain; an alkenyl or alkynyl group containing from 2 to 10 carbon atoms in linear or branched chain; one or several cycloalkyl, cycloalkenyl or cycloalkynyl group(s) containing from 3 to 10 carbon atoms in linear or branched chain; one or several aryl or heteroaryl group(s) containing from 3 to 10 carbon atoms per ring; an alkaryl or aralkyl group containing from 1 and 10 carbon atom(s), the words aryl and alkyl having the above definitions; an alkoxy, thioalkyl, sulfonylalkyl, aminoalkyl group containing from 1 to 10 carbon atom(s) in linear or branched chain; an alkoxyalkyl, alkylthioalkyl, alkylsulfonylalkyl, alkylaminoalkyl group containing from 1 and 10 carbon atoms in linear or branched chain; one or several heterocyclic group(s) containing from 5 to 10 carbon atoms per ring;
R'" represents H; an alkyl or haloalkyl group containing from 1 to 10 carbon atom(s) in a linear or branched chain;
and **in that**, optionally in a second step, deprotection of the obtained compound is carried out.

2. The method according to claim 1, wherein the group R of the cyclic peptide of the formula (IV) represents H; OH, SH; an alkyl or haloalkyl group containing from 1 to 10 carbon atom(s) in linear or branched chain; one or several aryl or heteroaryl group(s) containing from 3 to 10 carbon atoms per ring; an alkoxy, thioalkyl, sulfonylalkyl, aminoalkyl group containing from 1 to 10 carbon atom(s) in linear or branched chain; one or more heterocyclic group(s) containing from 5 to 10 carbon atoms per ring.

3. The method according to claim 2, wherein the group R of the cyclic peptide of the formula (IV) is selected from CH₂Cl, CH₂Br, CF₃, OH, O-CH₂-C₆H₆, NHOH, CH₂-S-CS-O-CH₂CH₃, CH₂CH₃, CO-CH₂CO₂-CH₂CH₃, CHOH-CH₃, CH(OTBDMS)CH₃.

4. The method according to any one of the preceding claims, wherein the group R' of the cyclic peptide of the formula (IV) represents H.

5. The method according to any one of the preceding claims, wherein the group R" of the compound of the formula (III) represents H, an alkyl or haloalkyl group containing from 1 to 10 carbon atom(s) in linear or branched chain; one or several aryl or heteroaryl group(s) containing from 3 to 10 carbon atoms per ring; an alkoxy, thioalkyl, sulfonylalkyl, aminoalkyl group containing from 1 to 10 carbon atoms in linear or branched chain; one or several heterocyclic group(s) containing from 5 to 10 carbon atoms per ring.

6. The method according to claim 5, wherein the group R" of the compound of the formula (III) is selected from CH₂Cl, CH₂Br, CF₃, O-CH₂-C₆H₆, CH₂CH₃, COCH₂CO₂Et and CH(OTBDMS)CH₃.

7. A synthesis intermediate compound of the formula (V): 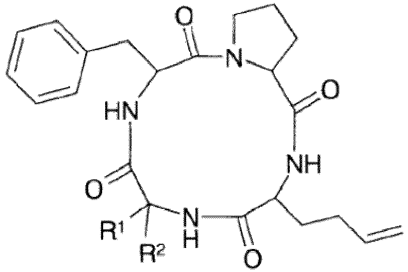 wherein R¹ and R² are as defined in any one of claims 1 to 3.

8. The compound according to claim 7, **characterized in that** at least one of R¹ and R² represents CH₂CΞCH, the other of R¹ and R² representing H, as appropriate.

9. A cyclic peptide of the formula (IV): 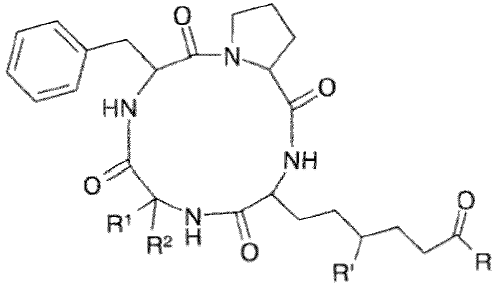 wherein,
R represents H; OH; SH; an amino group; an alkyl, haloalkyl or heteroalkyl group containing from 1 to 10 carbon atom(s) in linear or branched chain; an alkenyl or alkynyl group containing from 2 to 10 carbon atoms in linear or branched chain; one or several cycloalkyl, cycloalkenyl or cycloalkynyl group(s) containing from 3 to 10 carbon atoms in linear or branched chain; one or several aryl or heteroaryl group(s) containing from 3 to 10 carbon atoms per ring; an alkaryl or aralkyl group containing from 1 to 10 carbon atom(s), the words aryl and alkyl having the above definitions; an alkoxy, thioalkyl, sulfonylalkyl, aminoalkyl group containing from 1 to 10 carbon atom(s) in linear or branched chain; an alkoxyalkyl, alkylthioalkyl, alkylsulfonylalkyl, alkylaminoalkyl group containing from 1 and 30 carbon atoms in linear or branched chain; one or several heterocyclic group(s) containing from 5 to 10 carbon atoms per ring;
R' represents H or a xanthate group of the formula S-CS-O-R'" wherein R"' represents H, an alkyl or haloalkyl group containing from 1 to 10 carbon atom(s) in linear or branched chain;
at least one of R¹ and R² represents CH₂CΞCH; the other of R¹ and R² representing H, as appropriate.

10. The cyclic peptide according to claim 9, wherein R' represents H.

11. The cyclic peptide according to any one of claims 9 to 10, wherein R represents CHOH-CH₃.

12. The peptide according to any one of claims 9 to 11, for its use as an antiparasitic agent.

13. The peptide according to claim 12, for the treatment of malaria, toxoplasmosis, coccidiosis, cryptosporidiosis or neosporosis.

14. The peptide according to any one of claims 9 to 11, for the treatment of cancer.

15. A use of a peptide according to any one of claims 9 to 11, for treating an organ *ex vivo* prior to transplantation.
